Europäisches Patentamt

European Patent Office

Office européen des brevets

(19)

(11) EP 0 448 196 B1

(12) # EUROPEAN PATENT SPECIFICATION

(45) Date of publication and mention
of the grant of the patent:
**12.03.1997  Bulletin 1997/11**

(51) Int Cl.⁶: **G06F 19/00**
// G06F159:00

(21) Application number: **91300560.9**

(22) Date of filing: **24.01.1991**

(54) **Method and apparatus for spectral analysis of electrocardiographic signals**

Verfahren und Gerät zur Spektralanalyse von elektrokardiographischen Signalen

Méthode et appareil d'analyse spectrale de signaux électrocardiographiques

(84) Designated Contracting States:
**DE FR GB IT**

(30) Priority: **19.03.1990  US 496976**

(43) Date of publication of application:
**25.09.1991  Bulletin 1991/39**

(73) Proprietor: **DEL MAR AVIONICS**
**Irvine, California 92714-4878 (US)**

(72) Inventors:
 • **Kelen, George J.**
  **Staten Island, New York 10304 (US)**
 • **Henkin, Raphael**
  **Monarch Beach, California 92677 (US)**

(74) Representative: **Atkinson, Ralph et al**
**Fitzpatricks**
**Cardinal Court**
**23, Thomas More Street**
**London E1 9YY (GB)**

(56) References cited:
 • **PROCEEDINGS OF THE 2ND ANNUAL IEEE SYMP. ON COMPUTER-BASED MEDICAL SYSTEMS; IEEE COMPUTER SOCIETY PRESS, NEW YORK, US; 26 June 1989, MINNEAPOLIS, US pages 2 - 9; S.R.QUINT ET AL.: 'ASSESSING AUTONOMIC ACTIVITY FROM THE EKG RELATED TO SEIZURE ONSET DETECTION AND LOCALIZATION'**
 • **PROCEEDINGS OF COMPUTERS IN CARDIOLOGY; IEEE COMPUTER SOCIETY PRESS, NEW YORK, US; 25 September 1988, WASHINGTON, D.C., US pages 461 - 464; PENG-WIE HSIA ET AL.: 'DYNAMIC FREQUENCY SPECTRUM ANALYSIS: APPLICATION IN ORGANIZED AND DISORGANIZED ELECTROCARDIOGRAPHIC SIGNALS'**
 • **ICASSP-89 MULTIDIMENSIONAL SIGNAL PROCESSING AUDIO & ELECTROACOUSTICS; IEEE COMPUTER SOCIETY PRESS, NEW YORK, US; vol. 3, 23 May 1989, GLASGOW, SCOTLAND pages 1524 - 1527; R.M.S.S. ABEYSEKERA ET AL.: 'TIME-FREQUENCY DOMAIN FEATURES OF ECG SIGNALS: THEIR APPLICATION IN P WAVE DETECTION USING THE CROSS WIGNER-VILLE DISTRIBUTION'**

## Description

This invention relates to methods and devices for analysis of the electrical activity of the heart (Electrocardiography) and more particularly to the field known as High Resolution Electrocardiography, which is concerned with detecting abnormalities not apparent on conventional electrocardiograms. The specific and major purpose intended for the present invention is the clinical evaluation of medical patients for risk of life-threatening arrhythmias of the heart. However, both the method and apparatus are potentially suitable for research into and diagnosis of a wide variety of other disorders of cardiac electrical activity, and the analysis of other bio-electrical signals such as those recorded on an Electroencephalogram (EEG).

In order to fully comprehend the purpose and method of the present invention, it is necessary to be familiar with certain standard physiological and electrocardiographic (ECG) nomenclature. Relevant portions of that nomenclature are briefly summarized here, for the benefit of the reader not conversant with medical terminology.

The pumping action of the normal heart results from the orderly contraction of millions of individual muscle cells. Each heart beat is initiated by the spontaneous periodic activity of certain specialized cells of a structure known as the sino-atrial node. Activation of heart muscle cells is initiated by an electrical pacing signal generated in the sino-atrial node and propagated by specialized tissues known collectively as "the conducting system" of the heart. At the cellular level, the process of activation is known as "depolarization" since it involves transient changes in the electrical potential across the cell membrane mediated by the passage of ions. The process of recovery to the normal "resting" state is referred to as "repolarization".

The spread of the wavefront of electrical depolarization through the atria (or "primer pumping chambers") of the heart gives rise to the deflection on the electrocardiogram known as the "P" wave. Contraction of the main cardiac pumping chambers, or ventricles, is associated with a biphasic or multiphasic deflection of the ECG waveform known as the "QRS complex". Spread of the wavefront of electrical recovery (repolarization) through the ventricles gives rise to the "T" wave of the ECG. The time interval between the end of the QRS complex and the T wave, referred to as the "ST segment" is normally free from electrical activity, but may harbour small amplitude signals known as "late potentials" which extend out beyond the end of the QRS complex (viewed at ordinary magnification) into the ST segment. All of the components of ECG waveforms result from the synchronous spread of electrical signals associated with the activation or recovery of individual cardiac cells, across millions of such cells.

Over 50,000 people die annually in the USA of unexpected or sudden cardiac death, almost always from a catastrophic failure of normal electrical conduction within the heart known as ventricular fibrillation. Instead of the normal orderly and synchronous contraction of the heart muscle cells necessary for pumping of blood to occur, individual muscle fibres contract in a random and totally disorganised fashion. This arrhythmia (abnormal cardiac rhythm) frequently occurs in association with a myocardial infarction ("heart attack") either within the first minutes, hours, or sometimes months to years later, but is also the final common pathway for almost all forms of death from cardiac causes. Ventricular fibrillation is a terminal event because no blood at all is pumped and vital organs such as the brain die from lack of oxygen within minutes unless either the rhythm is corrected ("defibrillation") or the circulation is artificially supported by some other means such as CPR or cardio-pulmonary bypass ("heart-lung machine").

A frequent immediate predecessor to ventricular fibrillation is another arrhythmia known as ventricular tachycardia ("VT") during which, although some blood is pumped, the heart rate is usually much more rapid than the maximum rate at which the heart may function effectively as an efficient pump. This excessive heart rate frequently results in symptoms such as loss of consciousness or light-headedness. VT may spontaneously revert to a more normal heart rhythm ("non-sustained") or be prolonged for more than 30 seconds ("sustained"), in which latter case the development of symptoms or progression to ventricular fibrillation is much more common.

Despite the existence of a wide variety or more or less successful treatments such as drug therapy, surgery or implantable defibrillators for the prevention or correction of ventricular arrhythmias, their use is not without risk or expense of its own. Classical electrocardiography as practiced for several decades is unfortunately very poor at predicting who is likely to suffer such an event. Although it is known that increased risk is associated with certain states such as a severely damaged or dilated left ventricle (main pumping chamber of the heart), there remains an urgent need for a means of delineating those patients at significant risk, who might truly benefit from aggressive therapies, from amongst the vast majority of potential candidates in whom the risk of life threatening arrhythmia is low and for whom aggressive (or expensive) therapies may actually do more harm than good. Such an arrhythmia risk screening test should preferably be "non-invasive," i.e., not require breaking of the skin surface and free from risk and discomfort to the patient.

The most reliable method currently available for predicting likelihood of development of a lethal arrhythmia is a diagnostic procedure called "electrophysiological study" (EP) during which slender wires ("pacing catheters") are introduced through the skin into a large vein and advanced under X-ray guidance into the heart chambers themselves. Using a device attached to the wires, which generates electrical impulses similar to those produced by a pacemaker, the interior surface of the heart is subjected to electrical stimulation pulses in an attempt to induce VT. If it proves possible to induce ventricular tachycardia which is "monomorphic" (of one shape or type) and "sustained" (not spontaneously terminating) the patient is deemed to be much more likely to spontaneously develop a lethal rhythm disorder. Unfortunately, EP study is invasive, requires admitting the patient to a hospital, is often distressing to the patient who may need to be defibrillated out of a successfully induced arrhythmia, and is time, labour and cost intensive.

Among available non-invasive arrhymia risk assessment techniques, so-called "late potential analysis" has received most attention both in the literature and commercially. The patents of Simson, U.S. Patent No. 4,422,459, December 27, 1983, Netravali, U.S. Patent No. 4,458,691, July 10, 1984, and Strick, U.S. Patent No. 4,492,235, January 8, 1985, teach a method currently in widespread use on real-time ECGs. Many variants of the method have been advocated in the medical literature. Late potentials are signals of very low amplitude (less than 40 uV versus about 1 mV for the main ECG signal proper) and thus require employment of a special noise reduction technique known as "signal averaging" for them to be discernible above the background noise. Their presence beyond the end of the normal QRS complex is about 70-80% predictive for inducibility of sustained monomorphic VT at EP study and hence development of a serious spontaneous ventricular arrhythmia. However, late potential analysis cannot be used at all in the presence of certain relatively common types of ECG abnormality, and its specificity and sensitivity leave much to be desired.

The method of Simson teaches identification of late potentials by analysis in the time domain only, without any attempt to explicitly analyse their frequency, or spectral content. Bipolar ECG signals from three orthogonal surface leads are bidirectionally filtered and then algebraically summed into a single "vector magnitude" upon which certain characteristics of the terminal QRS signal are then measured. Late potentials are deemed to be present or absent depending upon the duration and amplitude of the terminal QRS region. As already noted, late potentials are not infrequently found (by time domain techniques) in perfectly normal, healthy individuals. The present invention teaches among other things, a novel method of distinguishing such "false positive" late potentials from those associated with serious arrhythmia risk, based upon frequency spectra, or their "spectrocardiographic" features.

In US Patent No. 4,680,708, July 14, 1987, Ambos, Cain & Sobel claim a frequency domain technique for late potential identification using Fourier analysis of a single, relatively long segment of ECG signal positioned over the terminal QRS region. Abnormality is allegedly characterised by presence of secondary high frequency peaks and an excess ratio of high frequencies to low frequencies.

In US Patent No. 4,883,065, one of the present inventors, Kelen, disclosed a novel system for the analysis of late potentials from long-term recordings of ECGs made on an ambulatory monitor, e.g. a Holter monitor.

Proceedings of the 2ND Annual IEEE Symposium on Computer-Based Medical Systems, IEEE Computer Society Press, New York, US, 26 June 1989, Minneapolis, US pages 2-9 describes a non-invasive method and apparatus for assessing an ECG signal for detecting alterations in autonomic activity.

The method comprises automatically or manually frequency analysing a relatively long ECG signal to identify heartbeat events during an interval spanning a period that a test subject is having a seizure such as an epileptic seizure. The relatively long ECG signal comprises, for example, 2048 heartbeats sequentially separated into 16 groups of 128 beats. The frequency analysis is directed to the heart period between successive beats. To do this, it is necessary to analyse the ECG signal to identify heart events, i.e. heartbeats, by mapping or demarcating the Q wave peak falling in the QRS complex of each heartbeat. Care has to taken that only the Q wave peaks are identified and no T wave peaks are allowed to compromise the string of events thus identified. The string of events identified in this way is then subjected to frequency analysis. Thus, this method extracts from said QRS complexes a point in time in which the heart is considered to have beat in order to be able to calculate variations in heart period between successive beats in order to detect alterations in autonomic activity of a test subject.

The present invention teaches spectral mapping of multiple overlapping ECG signal segments, spanning the whole QRS complex, with abnormality recognisable by visual features of three dimensional maps and computed parameters not disclosed or suggested by Cain or any other prior art. The novel method and apparatus of diagnosis via spectral feature analysis used in the present invention is applicable to the analysis of ECG signals recorded on Holter monitor tapes, as well as in real time.

An object of the present invention is to provide an ECG analysis method and apparatus by which it is possible to identify patients at significant risk of experiencing a serious ventricular arrhythmia.

Another object of the present invention is to provide a ventricular arrhythmia risk analysis method and apparatus that does not require invasion of the human body, puncturing of the patient's skin, admission to hospital, or significant risk, pain or discomfort to the patient.

Another object of the invention is to provide a method and apparatus for the calculation of numeric parameters which may be useful in distinguishing normal from abnormal heart function, in addition to the graphic display and hard copy generation of the spectral maps from which the numerical calculations are derived, thus providing both visual and qualitative and numerical qualitative methods for classification of recordings.

Another object of the invention is to provide a frequency analysis method and apparatus which in its standard default mode of operation performs, upon issue of a single command, analysis according to a fixed predefined stepwise signal processing protocol that results without further operator intervention in the generation of visual plots and numeric reports suitable for ventricular arrhythmia risk prediction. In this default routine mode of operation, the invention should be suitable for use by a technician.

Another object of the invention is to provide a frequency analysis method and apparatus having an operator interactive mode of operation whereby the individual steps of the analysis protocol can be customized from a menu, with sufficient flexibility and convenience so as to be suitable for use by researchers seeking to identify improved means of making electrocardiographic diagnoses based upon the visualization and measurement of the frequency characteristics of ECG signals as they change with time.

Various other objects and advantages of the present invention, and its most novel features, will become apparent to those skilled in the art by reading the accompanying specification and claims and studying the figures exemplifying the results of applying the invention to normal and abnormal subjects.

Briefly stated, the present invention comprehends a method and apparatus for performing frequency analysis upon relatively short, stepped but overlapping segments of ECG signals from one or more channels, in combination with pre-processing and post-processing steps appropriate to the intended purpose, to generate three dimensional maps reflecting the amplitude and time course of the various frequency components, or Fourier spectra, of said ECG signals. From the same frequency component or spectral data used to generate the maps, calculations are made of numeric parameters which quantify visually apparent morphological features of the maps, such as contour smoothness and number and position of frequency peaks. The three-dimensional maps, which may be plotted in several different orientations, are combined with a printout of the numeric parameters to generate a report from which both visual morphologic and quantitative assessments of abnormality can be made.

According to one aspect of the present invention, there is provided a method for frequency domain analysing an ECG signal, comprising the steps of:- locating within an ECG signal a time interval of interest; dividing said time interval of interest into a sequence of segments, or slices; multiplying each of said slices by a windowing function to form a windowed slice; performing a discrete fast Fourier transform (FFT) on each windowed slice, in which FFT the power spectral density (PSD) at a sequence of frequencies is calculated; and producing a numerical matrix or table of PSDs in which discrete frequencies head each column in said table and discrete times head each row of said table, thereby resulting in a matrix of PSDs for a vertically stacked array of time slices, characterised in that the method includes the steps of signal averaging a number of heartbeat cycles comprising said ECG signal prior to locating within the averaged ECG signal said time interval of interest, typically being the QRS region of said averaged ECG signal, and dividing said time interval of interest into a sequence of overlapping segments, or slices.

According to a second aspect of the present invention, there is provided an apparatus for analysing ECG signals to detect abnormalities of electrical conduction within the heart comprising: means for selectably storing a waveform representation of the amplitude-versus-time variation of an ECG signal; means for locating within said waveform a time interval of interest; means for dividing said time interval into a sequence of segments, or time slices; means for multiplying each of said slices by a windowing function to form a windowed slice; means for performing a discrete fast Fourier transform (FFT) on each windowed slice; means for calculating the power spectral density (PSD) at a series of frequencies in said FFT; and means for producing a numerical matrix or table of PSDs in which discrete frequencies head each column in said table and discrete times head each row of said table, thereby resulting in a matrix of PSDs for a vertically stacked array of time slices, characterised in that the apparatus includes means for signal averaging a number of heartbeat cycles comprising said ECG signal prior to locating within the averaged ECG signal a time interval of interest, typically being the QRS region of said averaged ECG signal, and said means for dividing divides the time interval of interest into a sequence of overlapping segments, or slices.

The foregoing and further features of the present invention will be more readily understood form the following description of a preferred embodiment, by way of example thereof, and with reference to the accompanying drawings, of which:-

Figure 1 is a block diagram of one embodiment of the apparatus for spectral analysis of electrocardiographic signals according to the present invention;
Figures 2 and 4 are spectral ECG plots generated by the apparatus of Figure 1 operated in its "default" mode;
Figures 5 to 7 are printed reports generated by the apparatus of Figure 1 corresponding to the spectral plots of Figures 2 to 4, respectively;
Figures 8 and 9 are spectral ECG plots generated by the apparatus of Figure 1, in which the default analysis protocol of the apparatus has been modified by operator intervention; and
Figure 10 is a schematic view of a table of intermediate values calculated by the apparatus of Figure 1.

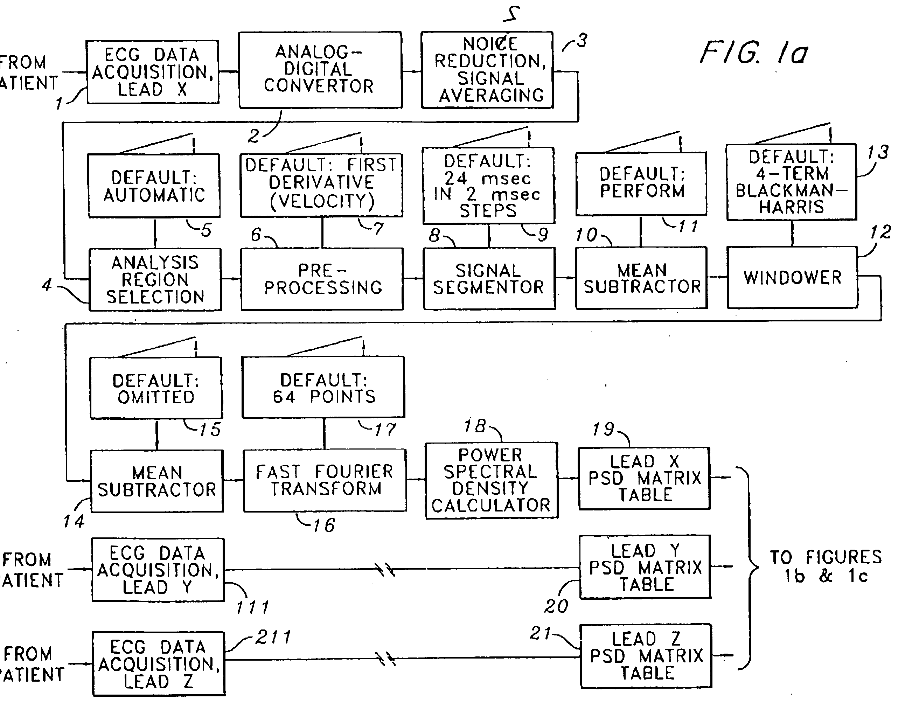

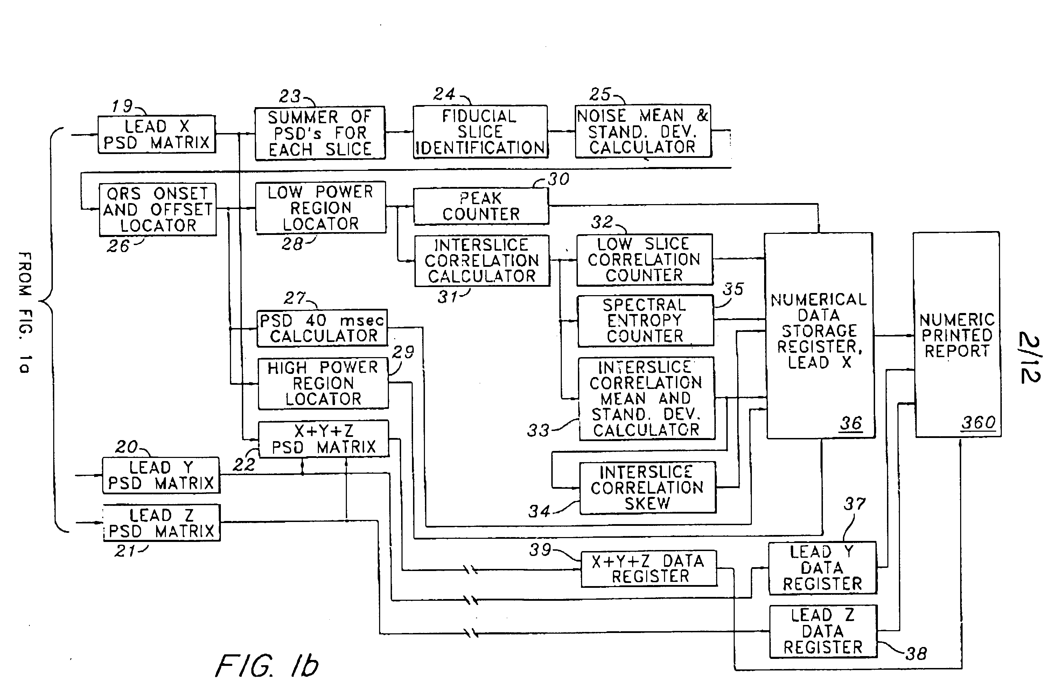

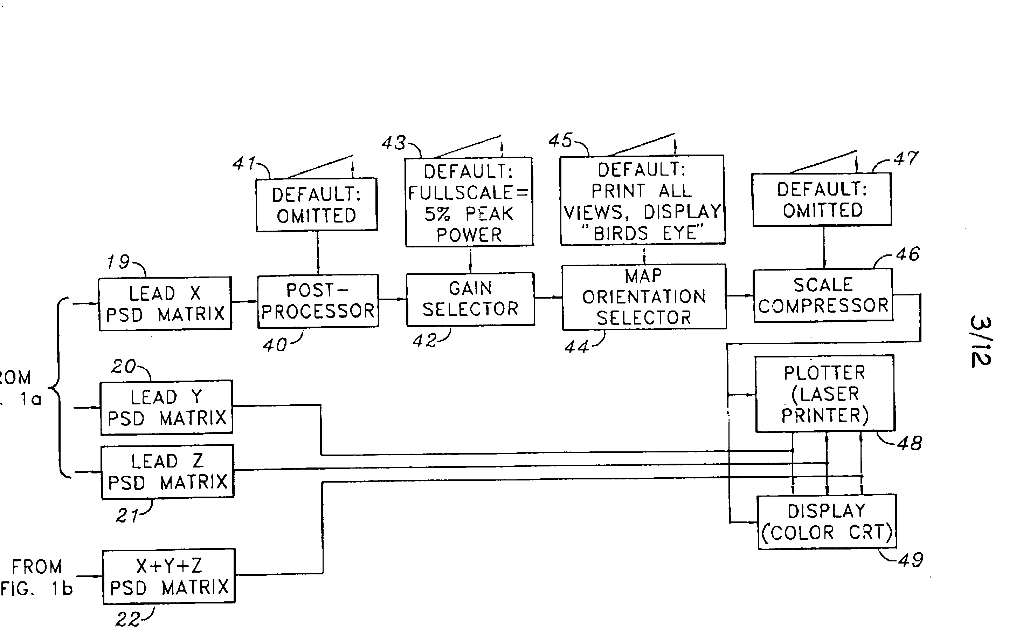

Figure 1 is a block diagram of a basic embodiment of an apparatus for the spectral analysis of electrocardiographic signals according to the present invention. Much of the apparatus of Figure 1 can be implemented as software modules running on a microprocessor system.

Those skilled in the art will recognise that the apparatus may be implemented with alternate means which perform the functions indicated for the various blocks of Figure 1.

Referring now to Figures 1A-1C, a simplified block diagram of the ECG frequency analysis system according to the present invention is shown. As shown in the Figures, most of the processing is performed separately but in identical fashion upon each of three channels (X, Y and Z) of signal averaged surface electrocardiogram. For simplicity, only the processing carried out on channel X is shown in the block diagram, except where there is a combination of data from more than one channel - specifically at blocks 22 and 39.

As shown in Figure 1A, block 1, three orthogonal channels (X, Y and Z) of bipolar electrocardiographic signal are acquired over a timespan of several hundred beats, either in real time by attachment of an appropriate isolated preamplifier directly to the patient, or from some other source, such as a previously recorded 24-hour Holter tape played back through an appropriate scanning device. At block 2, the acquired electrocardiographic signal is converted from its analog form to a digital form in conventional manner. At block 3, signal averaging is performed in order to reduce background noise to a level generally considered acceptable for conventional time domain late potential analysis, namely noise less than luV rms from 25-250 Hz. Generally some 200-500 heart beats need to be averaged to achieve the desired noise reduction. The actual frequency analysis claimed in this invention commences at block 4, with three channels of averaged ECG signal, digitized at typically 1000 Hz, with resolution of at least 1.5 uV per bit stored in computer memory or on some permanent storage medium.

In order to accomplish the dual purposes of providing both a standardized routine clinical and a flexible research application facility, analysis can be performed at user option in either of two modes. In automatic (default) mode the whole analysis is carried out upon issue of a single keystroke command, according to a predefined set of processing parameters, culminating in the printing of spectral maps and numeric printed reports. Otherwise, the user may access a menu prior to initiating the analysis, from which alternative analysis parameters in a wide range of permutations and combinations may be selected. Boxes on the figures where such optional user interaction is provided in this invention are identified with a switch symbol, indicating a capability for manual control. User override of the default parameters will henceforth be referred to as "manual" mode in this description.

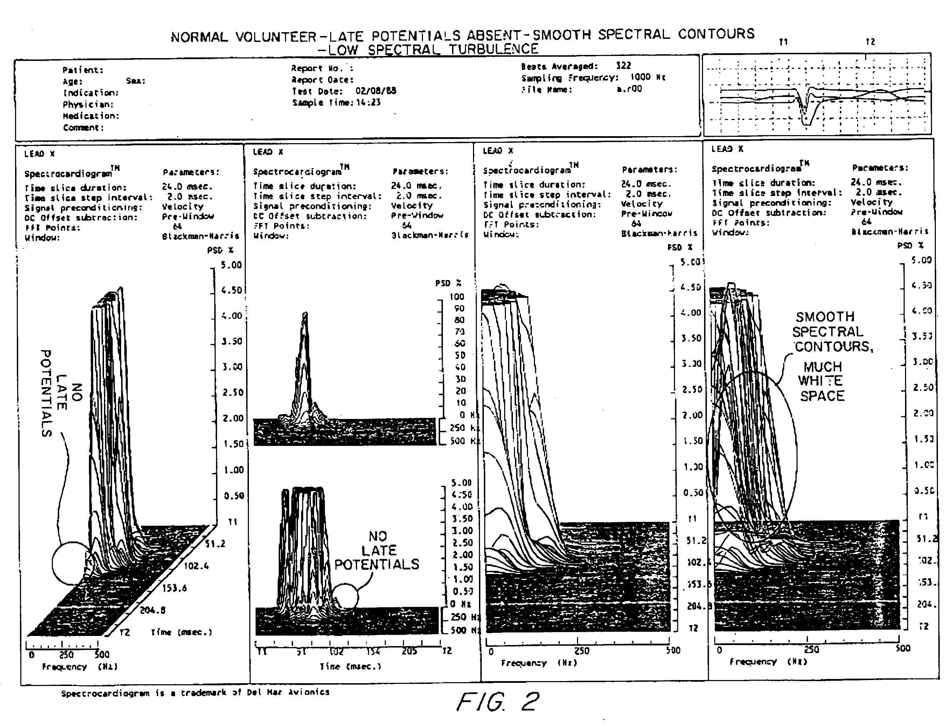

Returning now to Figure 1A, at block 4, the region of ECG signal over which the subsequent analysis is to be performed is determined. In default mode, the QRS region is first located by a computer algorithm (many suitable algorithms are known to those versed in the art) and the signal region commencing 25 msec before and terminating 125 msec after the QRS complex is demarcated. In manual mode as shown at block 5, the user indicates by using a mouse operated cursor the onset (beginning) and offset (end) of the region to be analyzed, as well as the orientation to be used for plotting of maps. Because of the three-dimensional nature of the frequency spectral plots as exemplified in Figures 2 - 4, 8 and 9, low amplitude waveforms of interest may be obscured "behind" larger ones. If interest is primarily in signals occurring before the QRS such as the P wave of the His-bundle, then the maps may be plotted with these structures " in front" so as not to be obscured by the much larger QRS waveform itself. On the other hand, if interest is primarily in signals such as late potentials which occur after the QRS (the default condition), then the maps are plotted in reverse direction, with later occurring structures plotted in front of the earlier occurring QRS.

The next step in analysis is signal pre-processing. At block 6 in default mode, the first derivative of the signal is calculated according to the following formula:

$$y[t] = (x[t+1] - x[t-1])/2 + (x[t+2] - x[t-2])/8$$

where y[t] is the new amplitude of the signal at sampling time t, x[t+1] the old amplitude of the next sampled point and so on. In manual mode, the user may elect, as depicted by block 7, to substitute for the processing at block 6 either no signal modification whatever or second derivative calculation according to the formula:

$$y[t] = x[t+1] + x[t-1] - 2(x[t])$$

using the same nomenclature as for the first derivative equation above. Since the indifferentiated ECG signal represents amplitude of the depolarization wavefront as a function of time, the first derivative corresponds to velocity, while the derivative is analogous to wavefront acceleration. Use of the first derivative in default mode minimizes the undesirable effect whereby a normal but sloping ST segment containing very high energy low frequency signal components obscures the presence of low amplitude late potentials in the spectral plots.

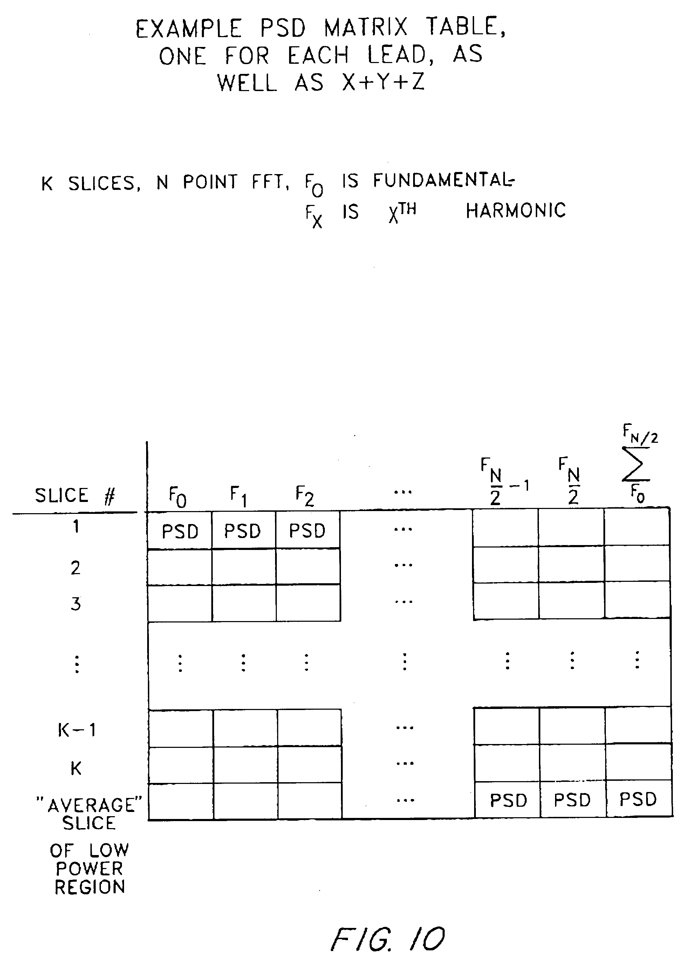

At block 8, the time spanning the signal region of interest is divided into overlapping segments or "time slices" of equal length according to the parameters of block 9. In default mode, slices are of 24-msec duration and each successive slice commences 2 msec later than its immediate predecessor. For example, if the signal region to be analyzed is 240 msec long, then there would be 1 + (240-24)/2 slices, or 109 slices submitted to further processing. In manual mode, segment length may range from 5 msec to 150 msec, while step interval may be selected from 1 msec. to 20 msec. Each time slice is then successively processed through blocks 10 to 18 until at block 19 a two dimensional table, Figure 10, is built in computer memory where each row represents the power spectral densities (PSD) at multiple frequency harmonics of a single time slice while each column contains the PSD's of all time slices at a single frequency, spanning the signal region of interest.

In default mode at block 10, the mean value of the signal is calculated for each time slice and subtracted from each data point within that slice so as to remove any DC offset. As shown in blocks 11 and 15 in manual mode, mean subtraction may be overridden altogether or delayed until after "w" at block 12.

In default mode at block 12, each time slice is multiplied by a 4-term Blackman-Harris window of equal length, in order to minimize spectral leakage and effects of edge discontinuities inherent in the Fast Fourier Transformation process of block 16. Rectangular, Hanning or Hamming window options may be substituted in manual mode as depicted in block 13.

In manual mode, windowing may be followed by mean subtraction to remove DC offset as shown by blocks 14 and 15, but in the default mode mean subtraction is done prior to windowing.

The next processing step is performance of a discrete Fast Fourier Transform (FFT) on each time slice, at block 16. The time slice data values are first moved to the beginning of a memory array which is then padded with zeroes to the length of the desired FFT. In default mode, a 64-point FFT is used but in manual mode 32 to 1024 points may be selected instead (block 17). For a default slice length of 24 msec represented by 25 data points and a 64 point FFT, points 26 to 64 are set to zero after which a double precision FFT is carried out.

At block 18, Power Spectral Densities are next calculated as the sum of the squares of the real and imaginary Fourier coefficients and for each successive time slice placed into the next row of the PSD matrix table at block 19.

The processing of blocks 8 to 18 is repeated for each time slice (and for each ECG lead) to build the PSD matrix tables of blocks 19,20 and 21 spanning the whole of the signal region of interest. These PSD tables are then used to generate spectral maps as per Figure 1C or to calculate further numeric parameters as per Figure 1B.

At Figure 1B, block 22, a fourth PSD matrix containing the mean values of X, Y and Z lead matrices is calculated. This "X + Y + Z Average" PSD table is henceforth displayed, plotted and used for numeric quantification in identical fashion to the original X, Y and Z leads.

As will become apparent from later discussion of the visual plots depicted in Figures 2 - 4, 8 and 9, we have discovered that the spectral maps of abnormal subjects are conspicuously "turbulent," with spectral contours less smooth than those of normal persons. The numeric calculations depicted in Figure 1B, blocks 23 to 35, quantify many of the differences between normal and abnormal maps so as to allow development of precise classification criteria. Each calculated parameter corresponds to a qualitative visual difference apparent from comparison of normal and abnormal spectral maps, as will be discussed below.

It will be recalled that each row of a lead's PSD matrix at block 19 contains the frequency analysis of a single time slice. At Figure 1B, block 23, an additional column referred to as the "total PSD column" is now added to the table for each ECG lead by summing the PSD at all frequency harmonics for each row to represent the sum total power spectral density at all frequences for each time slice. At block 24, the fiducial, or reference time slice of the QRS complex is identified as the row with the highest total PSD in the total PSD column just calculated.

Next, at block 25, the mean background noise value and its standard deviation are calculated by identifying the 40 msec region (21 consecutive time slices at the default step interval of 2 msec) within the total PSD column having the lowest average (total) PSD.

At block 26, the time slice rows corresponding to QRS onset and offset are next identified as the furthest rows, moving successively away from the QRS fiducial slice of block 24, having total PSD at least 5 standard deviations greater than the mean background noise level of box 25.

Also from the total PSD column calculated at block 23, a low power terminal region is defined as commencing at the first time slice following the fiducial QRS slice having total PSD less than 0.2% of the fiducial slice. This region is analogous to the late potential region of conventional time domain analysis. Thus, the total QRS complex demarcated at block 26 is divided at block 29 into a "high power" and at block 28 into a "low power terminal" region.

Meanwhile at block 27, the sum of the total PSD's over the terminal 40 msec of the QRS complex, demarcated at block 26, is calculated. We have discovered that the value of this parameter, known as " PSD40", is higher in normal subjects than in those with time domain late potential.

A further set of numerical computations is now performed separately upon the low power and high power QRS regions defined at blocks 28 and 29. For brevity, only the low power region calculations are elaborated here, since the processing performed upon the high power main QRS region is identical. One of the conspicuous differences between spectral maps from normal and abnormal subjects is the increased number of spectral peaks evident on the abnormal maps, especially at high frequencies. Accordingly at block 20, counts are made of spectral peaks within the low power terminal QRS region by six separate criteria. First, peaks are classified according to whether they occur above 250 Hz ("high frequency") or below 250 Hz ("low frequency"). Then, separately for high and low frequencies, counts are made of peaks occurring in the frequency axis along rows of the SD matrix table ("frequency axis peaks" ), in the time axis along columns of the PSD table (" temporal axis peaks" ) or in both axes simultaneously ("biaxial peaks"). We have found that subjects who have time domain late potentials and ventricular arrhythmias have higher peak counts than subjects with late potentials but no proneness to VT. Significantly, this characteristic of abnormals is true also for peaks counted in the main high power QRS region, as well as in the late potential region.

At block 31, a second additional column is calculated for each lead's PSD matrix table, containing the Pearson correlation coefficient of each row with the row immediately below it. This new column thus represents the degree to which the spectral characteristics of each time slice resemble the spectrum of the adjacent time slice. This column of inter-slice correlation coefficients is later plotted in one of the visual display options at Figure 1C, block 49, and yields as well several further numeric criteria of diagnostic importance.

At Figure 1B, block 32, the number of instances of an inter-slice correlation coefficient having a value less than 0.985 is counted. We have observed that normal subjects have very few instances of poorly correlated time slice pairs, whereas the incidence in patients prone to VT is much higher.

Block 33 depicts calculation of the mean and standard deviation of the correlation coefficients in the column calculated at block 31. The time slices of normal subjects have a higher mean correlation and lower standard deviation than abnormals.

At block 34, the skew of the distribution of correlation coefficients in the column calculated at block 31 is found by dividing the number of instances of correlation coefficient less than the mean value calculated at block 33 by the total number of slices in the low power region. In normal subjects, correlation coefficients are more evenly distributed around their mean than in abnormal subjects.

At block 35, a parameter we call "spectral entropy" is calculated to estimate the degree to which the frequency spectra of time slices differ from the average frequency spectrum of the region. Spectral entropy is calculated as follows. An additional row is added to the PSD matrix table to contain the calculated average PSD at each harmonic frequency of the rows representing the time slices of the low power region. The average correlation of each time slice row with this new row of average PSDs is next calculated and then subtracted from 1 to yield the spectral entropy statistic for the region. We have found that normal subjects have lower spectral entropy values than abnormals.

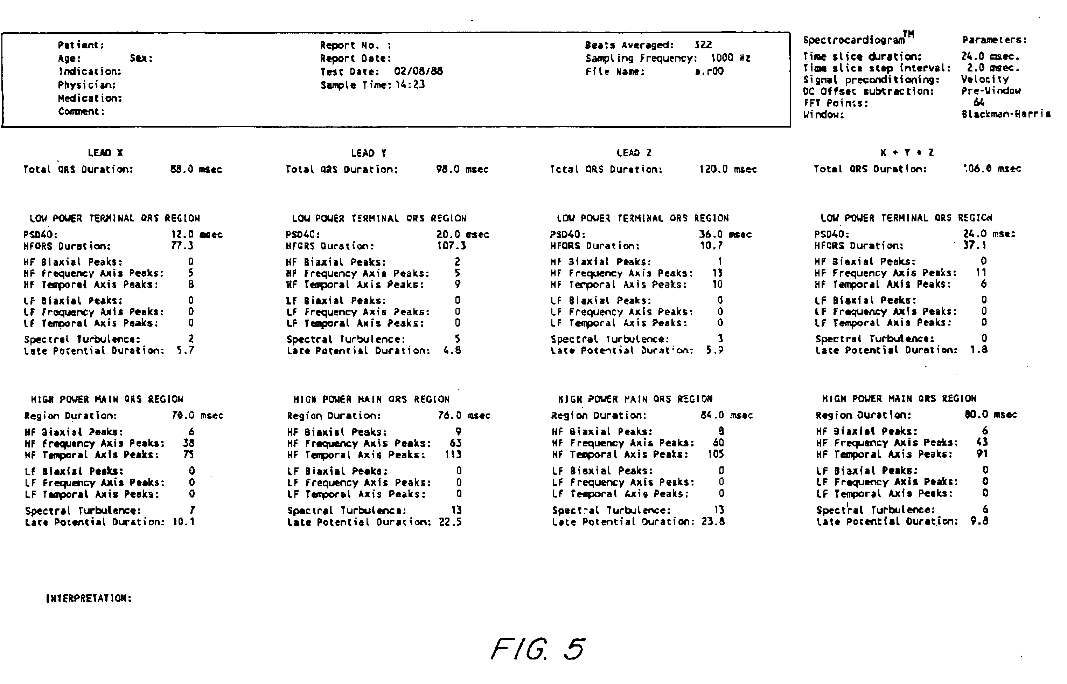

At block 36, the numeric parameters derived at blocks 27 to 35 are collected together to form the numeric parameters for lead X, which at block 40 is combined with identically derived numerics from leads Y, Z and the synthesized XYZ average matrix to make up the printed numeric report exemplified in Figures 5 to 7.

Returning now to blocks 19,20,21 and 22 representing the PSD matrix tables, Figure 1C illustrates how these tables are used to generate visual CRT displays and the printed spectral maps exemplified in figures 2 - 4, 8 and 9.

At Figure 1C, block 41 in manual mode, the user may optionally elect to display or plot not the values actually contained in the PSD matrix tables but rather their first or second derivatives with respect to time. In default mode, no calculations or changes are made at block 40. If first derivative ("velocity") post-processing is selected at block 41, each time slice row is subtracted from the row above it thus representing the change in PSD at each slice step. If second derivative post-processing ("acceleration") is selected, then each time slice row is subtracted from the mean of the row immediately above and row immediately below it. These optional post-processing steps accentuate some of the visual differences between the maps of normals and abnormals.

At block 42, the display and plotting gain is determined. The gain used in default mode is 20, such that full scale corresponds to 5% peak PSD, a value found by experimentation to yield good resolution of the visual features useful in clinical diagnosis. In manual mode the user may, at block 43, select gains from .01 to 1000.

At block 44, the orientation of spectral maps to be drawn and/or displayed is determined. Inspection of Figures 2 - 4, 8 and 9 will disclose the utility of being able to view these three dimensional structures from multiple angles, since no single view is ideal for looking at all features of potential interest. In default mode, all available views are automatically printed, but this may be overridden in manual mode at block 45 to select single or restricted combinations of views.

In addition to gain and view selection, the invention provides for optional nonlinear scale compression in manual mode prior to display or plotting. In default mode, PSD values are used, but as depicted at block 47, in manual mode, the user may select Power (square root of PSD) or logarithmic (dB) representation of the spectral data instead.

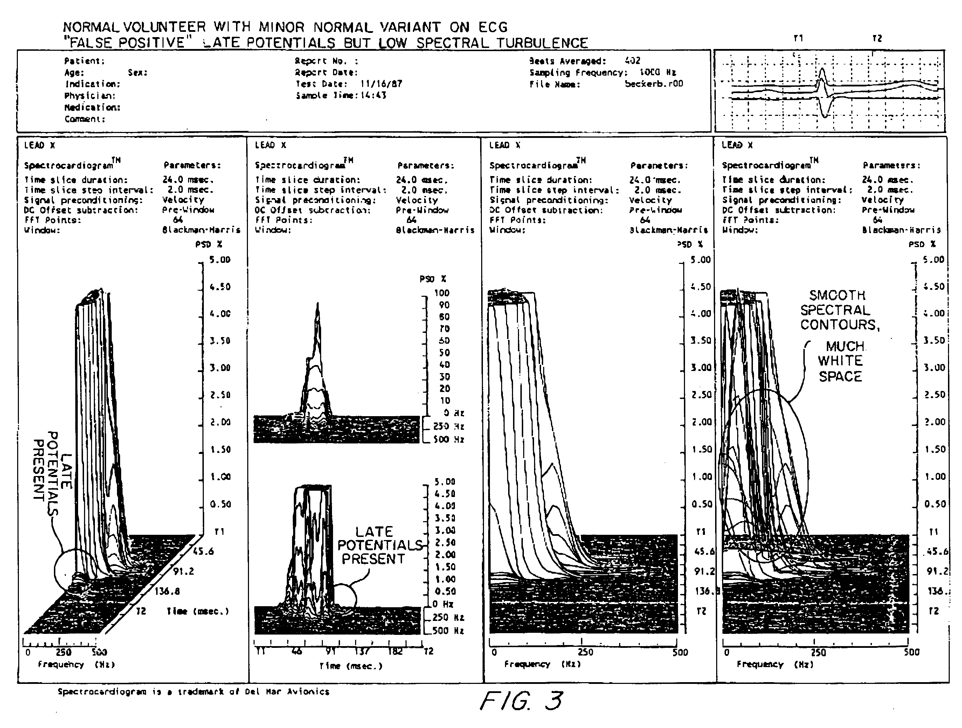

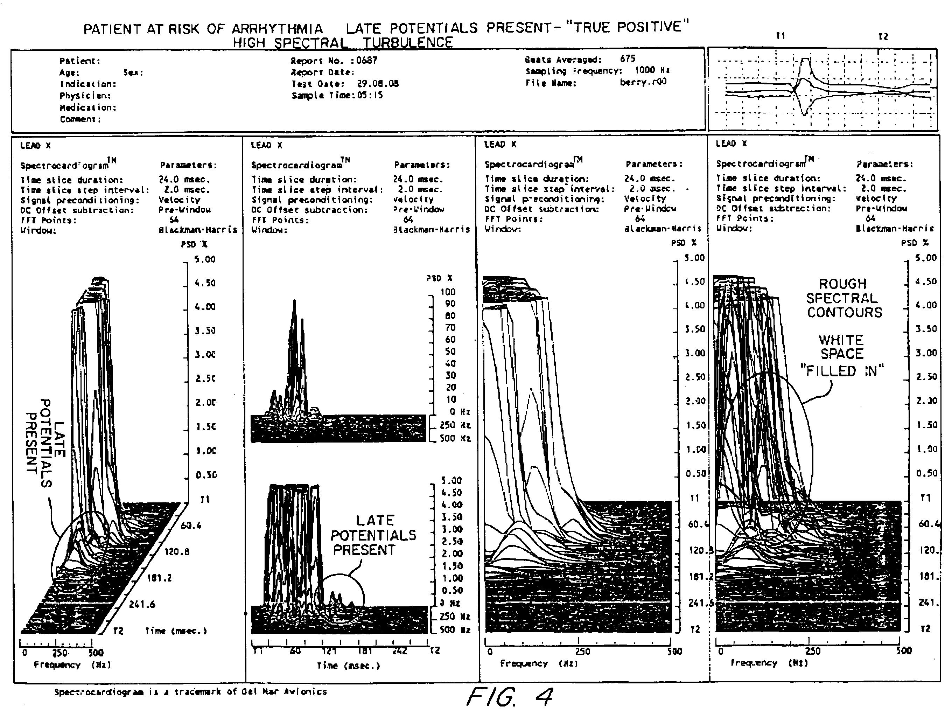

Referring now to Figures 2-4, some specific examples of the benefits of the invention are shown by recordings made from actual patients. Figures 2 and 3 are both from normal healthy volunteers with no heart disease and no proneness to ventricular arrhythmia. Figure 4 is from a patient who has suffered a heart attack and has had episodes of life threatening arrhythmia. Although not at risk, the subject of Figure 3 ha a common minor normal variant of the electrocardiogram known as " Incomplete Right Bundle Branch Block" which results in the end portion f the QRS complex being slightly slurred. Such slurring results in a "false positive" late potential, and the subject of figure 3 would be classified as " at risk" on the basis of a test for presence of late potentials. The late potential signals are indicated by arrows on Figures 3 and 4. Note how both patients have low amplitude signals extending out beyond the end of the QRS complex. However, in Figure 3 ("false positive"), the spectral contours of the late potentials are smooth, whereas in figure 4 ("true positive") the spectral contours are turbulent and disorganized. Note also how much more turbulent the spectral contours appear throughout the whole QRS complex (not just the late potential region) in the "true positive" patient in Figure 4.

The above illustrates one instance of how the present invention provides a means for determining the correct risk classification of a patient who would be misdiagnosed as "at risk" by techniques of the prior art.

Figure 5 is the numeric report of a healthy volunteer generated from the spectral plot shown in Figure 2.

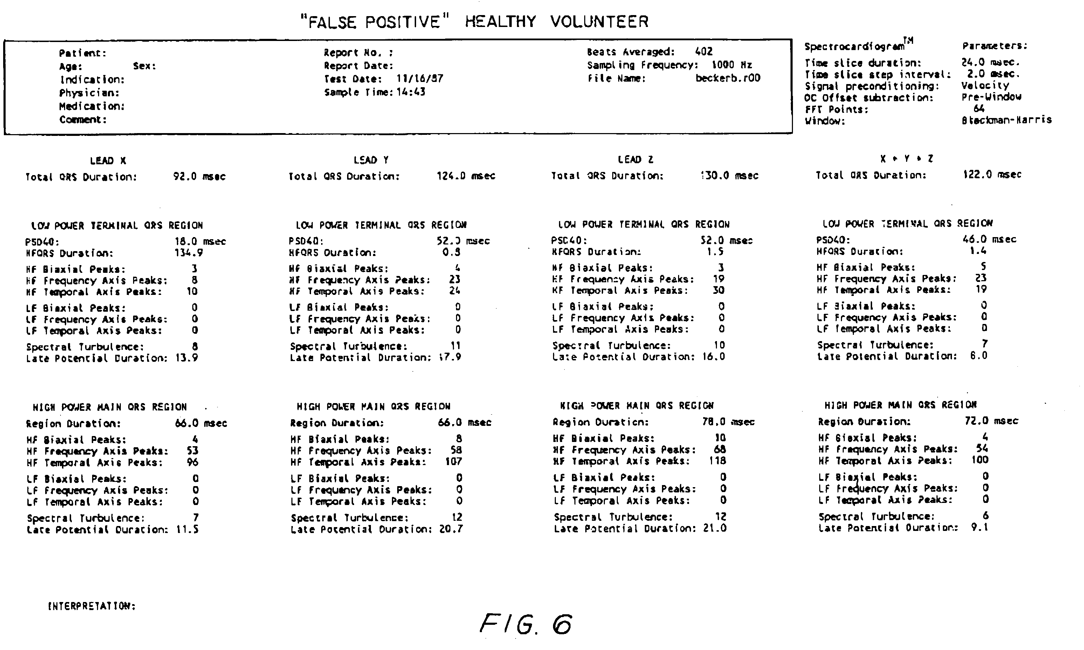

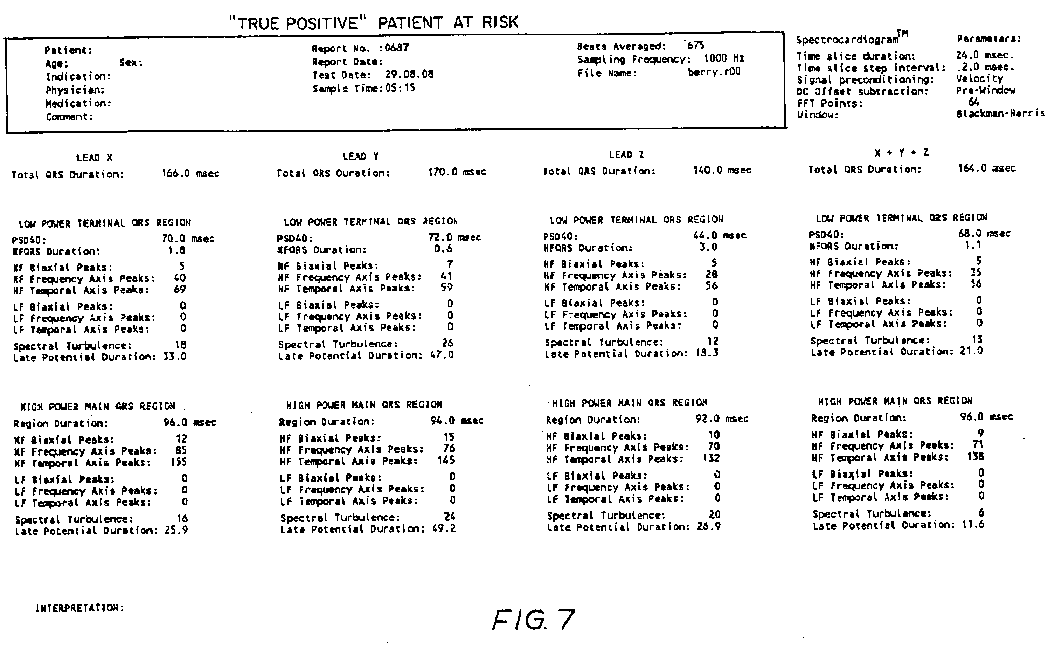

Figure 6 is a numeric report generated from the "false positive" patient whose spectral plots are shown in figure 3, while Figure 7 is from the "true positive" patient of Figure 4. Note how the differences visually observed in the spectral plots result in correspondingly different calculated numeric data, enabling specific numeric criteria of abnormality to be developed for diagnostic purposes.

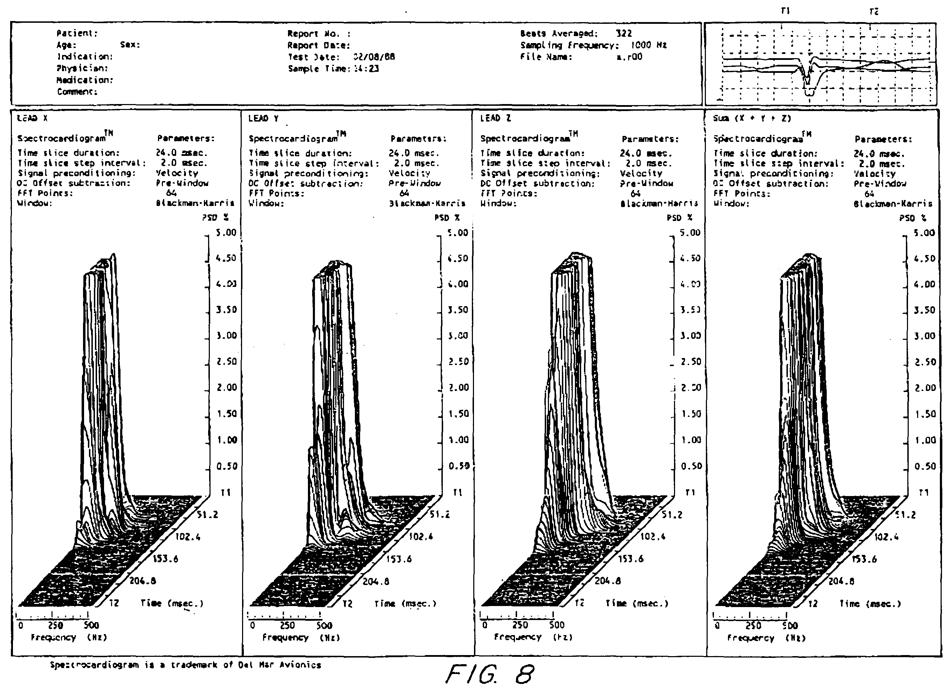

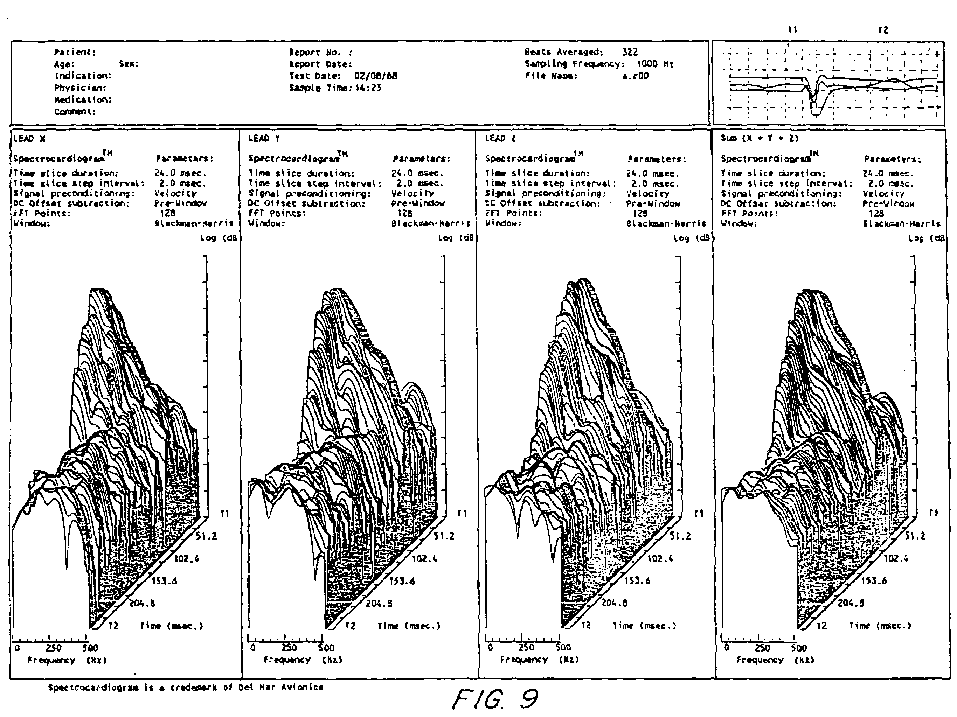

Figures 8 and 9 are spectral plots generated by the apparatus of figure 1, from the same raw data from the healthy volunteer used to generate Figures 2 and 5. However, in Figures 8 and 9, the default analysis protocol of the apparatus has been modified by operator intervention, as follows.

Figure 8 displays on a linear vertical, or ordinate scale, the amplitude of power spectral densities of the ECG waveform from three orthogonal lead sets, X, Y and Z, plus a fourth spectrocardiogram of the sum of the signals from the X, Y and Z leads.

Figure 9 shown the power spectral densities of the same lead configuration as Figure 8, but with the power spectral densities displayed on a logarithmic (db) scale, rather than a linear scale.

It should be recognized that Figures 8 and 9 are just two examples from a very large number of variations in display format which are made possible by the novel method and apparatus of the present invention. Some of these variations may prove useful in the detection of other abnormalities in ECG's or other bioelectrical signals, in addition to the displays of Figures 3 and 4 which clearly demonstrate the power of the present invention to distinguish between false positives and patients who are truly at risk.

## Claims

1. A method for frequency domain analysing an ECG signal, comprising the steps of:- locating within an ECG signal a time interval of interest; dividing said time interval of interest into a sequence of segments, or slices; multiplying each of said slices by a windowing function to form a windowed slice; performing a discrete fast Fourier transform (FFT) on each windowed slice, in which FFT the power spectral density (PSD) at a sequence of frequencies is calculated; and producing a numerical matrix or table of PSDs in which discrete frequencies head each column in said table and discrete times head each row of said table, thereby resulting in a matrix of PSDs for a vertically stacked array of time slices, characterised in that the method includes the steps of signal averaging a number of heartbeat cycles comprising said ECG signal prior to locating within the averaged ECG signal said time interval of interest, typically being the QRS region of said averaged ECG signal, and dividing said time interval of interest into a sequence of overlapping segments, or slices.

2. A method as claimed in claim 1, characterised in that it includes the further steps of:- calculating a mean value of the amplitude of each time slice prior to performing said FFT; and subtracting said mean value of each time slice from each data point within that slice thereby removing a D.C. offset in said time slice.

3. A method as claimed in claim 1 or claim 2, characterised in that it includes the further steps of:- performing the same steps on at least one additional ECG signal obtained from a patient simultaneously with said first ECG signal, thereby obtaining a plurality of N ECG signals and N PSD matrices; and summing said mean values of said N PSD matrices, thereby forming a (N+1)th average PSD matrix.

4. A method as claimed in any preceding claim, characterised in that it includes the further steps of:- summing each PSD value in a time slice row to form a total PSD sum, thereby forming a total PSD column; and comparing each total PSD value in each vertical position in said PSD column with each other PSD value to determine that time slice row having the highest total PSD value, and designating said time slice row as the fiducial, or reference time slice of said QRS complex.

5. A method as claimed in claim 4, characterised in that it includes the further steps of:- locating the onset and offset of the QRS portion of said ECG signal; calculating the mean background noise value and standard deviation of said ECG signal represented by said total PSD matrix by identifying a given time interval, represented by a pre-determined number of consecutive time slices within said PSD matrix having the lowest average total PSD; and identifying that uppermost (earliest) row and lowermost (latest) row away from said QRS fiducial slice having a total PSD at least N1 standard deviations greater than said mean background noise.

6. A method as claimed in claim 5, characterised in that it includes the further step of:- calculating the sum of the total PSDs over a terminal interval T1 of said QRS complex.

7. A method as claimed in claim 6, characterised in that it includes the further steps of:- dividing said QRS region of said PSD matrix into a low power terminal region, and a high power region, said low power terminal region being defined as commencing at the first time slice following said fiducial QRS slice having a total PSD less than a percentage P1 of said fiducial slice, and ending at the termination of the QRS complex, and said high power region of said QRS region being defined as the remainder of said QRS region of said PSD matrix.

8. An apparatus for analysing ECG signals to detect abnormalities of electrical conduction within the heart comprising: means for selectably storing a waveform representation of the amplitude-versus-time variation of an ECG signal; means (4) for locating within said waveform a time interval of interest; means (8) for dividing said time interval into a sequence of segments, or time slices; means (12) for multiplying each of said slices by a windowing function to form a windowed slice; means (16) for performing a discrete fast Fourier transform (FFT) on each windowed slice; means (18) for calculating the power spectral density (PSD) at a series of frequencies in said FFT; and means (19) for producing a numerical matrix or table of PSDs in which discrete frequencies head each column in said table and discrete times head each row of said table, thereby resulting in a matrix of PSDs for a vertically stacked array of time slices, characterised in that the apparatus includes means (3) for signal averaging a number of heartbeat cycles comprising said ECG signal prior to locating within the averaged ECG signal a time interval of interest, typically being the QRS region of said averaged ECG signal, and said means (8) for dividing the time interval of interest into a sequence of overlapping segments, or slices.

9. An apparatus as claimed in claim 8, characterised in that it includes means for comparing said table of PSDs with corresponding tables obtained from normal and abnormal test subjects.

10. An apparatus as claimed in claim 8 or claim 9, characterised in that it includes: means (6) for calculating the mean value of the time-domain amplitude of each of said time slices, prior to performing said windowing; and means (10,14) for subtracting said mean time-domain value of each slice from each data point value within that slice, thereby removing a D.C. offset in said time slice.

11. An apparatus as claimed in any one of claims 8 to 10, characterised in that said apparatus is capable of performing the same functions on a plurality of separate ECG channels.

12. An apparatus as claimed in claim 11, characterised in that it includes: means (23) for summing each PSD value in a time slice row to form a total PSD sum, thereby forming a total PSD column; and means (24) for comparing said total PSD value in each vertical position in said PSD column with each other PSD value and determining that time slice row having the highest total PSD value, thereby determining a fiducial or reference time slice of the QRS complex of said ECG waveforms.

13. An apparatus as claimed in claim 12, characterised in that it includes: means (26) for locating the onset and offset of said QRS portion of said ECG signal; means (27) for calculating the sum of the total PSDs over a terminal interval T1 of said QRS complex; means (28,29) for segmenting said QRS portion of said PSD matrix into a low power terminal portion and a high power portion, said low power terminal portion being defined as commencing at the first time slice following said QRS slice having a total PSD less than a percentage P1 of said fiducial slice, and ending at the end of said matrix, and said high power portion of said QRS portion being defined as the remainder of said QRS portion of said PSD matrix.

14. An apparatus as claimed in claim 13, characterised in that it includes: means for further separate processing including means for counting those peaks for maxima occurring along the frequency axis or rows of said PSD matrix table (frequency axis peaks), those peaks or maxima occurring along the time axis or columns of said PSD matrix table (temporal axis peaks), and those peaks occurring in both axes simultaneously (biaxial peaks).

15. An apparatus as claimed in claim 14, characterised in that it includes: means (32) for calculating for each said PSD matrix table the Pearson correlation coefficient of each row with the row immediately below it (inter-slice correlation coefficient) and storing for display an additional column of numbers corresponding to said correlation coefficients.

16. An apparatus as claimed in claim 15, characterised in that it includes: means (35) for calculating and storing for display a number called spectral entropy by adding an additional row to said PSD matrix table, said additional row containing the calculated average PSD of each harmonic frequency of selected rows of said PSD calculating the average correlation of each time slice row with the new row of average PSDs and subtracting said average correlation from 1.

17. An apparatus as claimed in any one of claims 8 to 16, characterised in that it includes: means (48,49) for displaying tables of numbers derived from numbers in said matrix tables in three-dimensional-like contour plots with the amplitude of said spectral components versus time and frequency.

## Patentansprüche

1. Verfahren für einen Frequenzbereich zum Analysieren eines EKG-Signals, bestehend aus folgenden Schritten: Feststellen eines Zeitintervalls, innerhalb eines EKG-Signals, das von Interesse ist; Teilen des genannten Intervalls, das von Interesse ist, in eine Segmentfolge oder Zeitscheiben; Multiplikation der einzelnen genannten Zeitscheiben durch eine Fensterfunktion, um eine Fensterscheibe zu bilden; Anwendung eines FFT-Verfahrens an jeder Fensterscheibe, bei der durch das FFT-Verfahren die spektrale Leistungsdichte (PSD) einer Frequenzfolge berechnet wird und Erstellen einer numerischen PSD-Matrix oder -Tabelle, in der diskrete Frequenzen als Titel für jede Spalte in der genannten Tabelle verwendet werden und diskrete Zeiten als Titel für jede Reihe in der genannten Tabelle, so daß sich eine PSD-Matrix für eine senkrecht geschichtete Zeitscheibengruppe ergibt, dadurch gekennzeichnet, daß das Verfahren Schritte wie die Signal-Mittelwertbildung von Herzschlagfrequenzen einschließt, die das genannte EKG-Signal enthalten, bevor im EKG-Signal, für das die Mittelwertbildung gilt, das genannte Zeitintervall von Interesse festgestellt wird, wobei es sich typisch um den QRS-Bereich des genannten EKG-Mittelwertsignals handelt und Teilen des genannten Zeitintervalls, das von Interesse ist, in eine Folge überlappender Zeitsegmente oder -scheiben.

2. Verfahren nach Anspruch 1, dadurch gekennzeichnet, daß es die folgenden weiteren Schritte enthält: Berechnen eines Mittelwertes für die Amplitude einer jeden Zeitscheibe vor Ausführen des genannten FFT-Verfahrens und Abzug des genannten Mittelwertes von jeder Zeitscheibe von jedem Datenpunkt innerhalb der genannten Scheibe, wobei eine Gleichstromversetzung in der genannten Zeitscheibe entfernt wird.

3. Verfahren nach Anspruch 1 oder 2, dadurch gekennzeichnet, daß folgende weitere Schritte eingeschlossen sind: Ausführen der gleichen Schritte an mindestens einem zusätzlichen EKG-Signal von einem Patienten, gleichzeitig mit dem ersten genannten EKG-Signal, so daß mehrere, N, EKG-Signale und N PSD-Matrizen erhalten werden und Summieren der genannten Mittelwerte der genannten N PSD-Matrizen, so daß sich eine (N+1). PSD-Durchschnittsmatrix ergibt.

4. Verfahren nach einem der vorstehenden Ansprüche, dadurch gekennzeichnet, daß es folgende weitere Schritte enthält: Summieren der einzelnen PSD-Werte in einer Zeitscheibenreihe, um eine PSD-Gesamtsumme zu bilden, so daß eine PSD-Gesamtspalte gebildet wird und Vergleich der einzelnen PSD-Gesamtwerte in jeder senkrechten Position in der genannten PSD-Spalte mit jedem anderen PSD-Wert, um die Zeitscheibenreihe zu bestimmen, die den höchsten PSD-Gesamtwert aufweist und Bezeichnen der genannten Zeitscheibenreihe als Bezugs- oder Referenz-Zeitscheibe des genannten QRS-Komplexes.

5. Verfahren nach Anspruch 4, dadurch gekennzeichnet, daß es folgende weitere Schritte einschließt: Feststellen von Anfang und Ende des QRS-Teils des genannten EKG-Signals; Berechnen des mittleren Geräuschpegelwertes und der Standardabweichung des genannten EKG-Signals, das von der genannten PSD-Gesamtmatrix dargestellt wird, durch Feststellen eines bestimmten Zeitintervalls, dargestellt von einer vorbestimmten Anzahl aufeinanderfolgender Zeitscheiben innerhalb der genannten PSD-Matrix, die die niedrigste Gesamtdurchschnitts-PSD aufweist und Feststellen der obersten (frühesten) Reihe und untersten (letzten) Reihe von der genannten QRS-Bezugsscheibe weg mit einer PSD-Gesamtsumme von mindestens N1 Standardabweichungen mehr als der genannte mittlere Geräuschpegel.

6. Verfahren nach Anspruch 5, dadurch gekennzeichnet, daß es folgende weitere Schritte enthält: Berechnen der PSD-Gesamtsumme in einem Endintervall T1 des genannten QRS-Komplexes.

7. Verfahren nach Anspruch 6, dadurch gekennzeichnet, daß es folgende weitere Schritte enthält: Teilen des genannten QRS-Bereichs der genannten PSD-Matrix in einen schwachen Stromendbereich und einen starken Strombereich, wobei der schwache Stromendbereich so definiert wird, daß er an der ersten Zeitscheibe beginnt, die auf die genannte QRS-Bezugsscheibe folgt, mit einer PSD-Gesamtsumme von unter einem Prozentsatz P1 der genannten Bezugsscheibe und endend am Ende des QRS-Komplexes, wobei der genannte starke Strombereich des genannten QRS-Bereichs als Rest des genannten QRS-Bereichs der genannten PSD-Matrix definiert wird.

8. Vorrichtung zum Analysieren eines EKG-Signals zum Feststellen von Unregelmäßigkeiten in der Stromleitung innerhalb des Herzens, bestehend aus: Einem Mittel zur wählbaren Speicherung einer Wellenform-Darstellung von Schwankungen in der Amplitude im Vergleich zur Zeit eines EKG-Signals; einem Mittel (4) zum Feststellen der genannten Wellenform in einem Zeitintervall, das von Interesse ist; einem Mittel (8) zum Teilen des genannten Zeitintervalls in einer Segmentfolge oder Zeitscheiben; einem Mittel (12) zum Multiplizieren der einzelnen genannten Scheiben durch eine Fensterfunktion, um eine Fensterscheibe zu bilden; einem Mittel (16) zum Anwenden eines diskreten FFT-Verfahrens an jeder Fensterscheibe; einem Mittel (18) zum Berechnen der spektralen Leistungsdichte (PSD) bei mehreren Frequenzen im genannten FFT sowie einem Mittel (19) zum Erstellen einer numerischen PSD-Matrix oder -Tabelle, in der diskrete Frequenzen als Titel über jeder Spalte in der genannten Tabelle und diskrete Zeiten als Titel vor jeder Reihe der genannten Tabelle verwendet werden, wodurch sich eine PSD-Matrix für eine geschichtete Zeitscheiben-Gruppe ergibt, dadurch gekennzeichnet, daß die Vorrichtung ein Mittel (3) zum Berechnen des Signalmittelwertes von mehreren Herzschlagfrequenzen einschließt, bestehend aus dem genannten EKG-Signal vor Feststellen eines Zeitintervalls von Interesse im EKG-Mittelwertsignal, wobei es sich typisch um den QRS-Bereich des genannten EKG-Mittelwertsignals handelt und einem genannten Mittel (8) zum Teilen der Zeitintervall-Teile, die von Interesse sind, in eine überlappende Segmentfolge oder Scheiben.

9. Vorrichtung nach Anspruch 8, dadurch gekennzeichnet, daß sie ein Mittel zum Vergleichen der genannten PSD-Tabelle mit entsprechenden Tabellen von normalen und pathologischen Probanden einschließt.

10. Vorrichtung nach Anspruch 8 oder 9, dadurch gekennzeichnet, daß sie einschließt: Ein Mittel (6) zum Berechnen des Mittelwertes der Zeitbereichsamplitude für jede der genannten Zeitscheiben vor der genannten Fensterfunktion und ein Mittel (10,14) zum Abziehen des genannten mittleren Zeitbereichswertes für jede Scheibe von jedem Datenpunktwert in der Scheibe, so daß eine Gleichstrom-Versetzung in der genannten Zeitscheibe entfernt wird.

11. Vorrichtung laut Anspruch 8 bis 10, dadurch gekennzeichnet, daß die genannte Vorrichtung die gleichen Funktionen an mehreren getrennten EKG-Kanälen ausführen kann.

12. Vorrichtung nach Anspruch 11, dadurch gekennzeichnet, daß sie folgendes einschließt: Ein Mittel (23) zum Summieren der einzelnen PSD-Werte in einer Zeitscheiben-Reihe, um eine PSD-Gesamtsumme zu bilden, so daß eine PSD-Gesamtspalte entsteht und ein Mittel (24) zum Vergleichen des genannten PSD-Gesamtwertes in jeder senkrechten Position in der genannten PSD-Spalte mit jedem anderen PSD-Wert und die Bestimmung, daß der Zeitscheiben-Reihe, die den höchsten PSD-Gesamtwert hat und damit die Bestimmung einer Bezugs- oder Referenz-Zeitscheibe im QRS-Komplex der genannten EKG-Wellenformen.

13. Vorrichtung nach Anspruch 12, dadurch gekennzeichnet, daß sie einschließt: ein Mittel (26) zum Feststellen von Anfang und Ende des genannten QRS-Teils des genannten EKG-Signals; ein Mittel (27) zum Berechnen der PSD-Gesamtsumme in einem Endintervall T1 des genannten QRS-Komplexes; ein Mittel (28, 29) zum Segmentieren des genannten QRS-Teils der genannten PSD-Matrix in einen Endteil mit schwachem Strom und einen Teil mit starkem Strom, wobei der genannte Endteil mit schwachem Strom so definiert wird, daß er an der ersten Zeitscheibe nach der genannten QRS-Scheibe beginnt, die einen PSD-Gesamtwert von unter einem Prozentsatz P1 der genannten Bezugsscheibe hat und endend am Ende der genannten Matrix, wobei der genannte starke Stromteil des genannten QRS-Teils als der Rest des genannten QRS-Teils der genannten PSD-Matrix definiert wird.

14. Vorrichtung nach Anspruch 13, dadurch gekennzeichnet, daß sie einschließt: Ein Mittel zum weiteren getrennten Verarbeiten einschließlich einem Mittel zum Zählen von Höchstspitzen, die auf der Frequenzachse oder in Reihen der genannten PSD-Matrixtabelle (Frequenzachsen-Spitzen) auftreten, wobei diese Spitzen oder Höchstwerte auf der Zeitachse oder in Spalten der genannten PSD-Matrixtabelle auftreten (Zeitachsen-Spitzen) und die Spitzen auf beiden Achsen gleichzeitig auftreten (zweiachsige Spitzen).

15. Vorrichtung nach Anspruch 14, dadurch gekennzeichnet, daß sie einschließt: Ein Mittel (32) zum Berechnen der einzelnen genannten PSD-Matrixtabelle, des Pearsonschen Korrelationskoeffizienten für jede Reihe mit der direkt darunter befindlichen Reihe (Korrelationskoeffizient zwischen den Scheiben) und zum Speichern zur Anzeige einer zusätzlichen Zahlenspalte entsprechend dem genannten Korrelationskoeffizienten.

16. Vorrichtung nach Anspruch 15, dadurch gekennzeichnet, daß sie einschließt: ein Mittel (35) zum Berechnen und Speichern einer sogenannten Spektralentropie-Zahl für die Anzeige durch Addieren einer zusätzlichen Reihe der genannten PSD-Matrixtabelle, wobei die zusätzliche Reihe den berechneten PSD-Durchschnitt für jede Oberwellenfrequenz der gewählten Reihen der genannten PSD enthält, wobei die durchschnittliche Korrelation einer jeden Zeitscheibenreihe mit der neuen Reihe PSD-Durchschnittswerte berechnet wird und Abziehen der genannten Durchschnittskorrelation von 1.

17. Vorrichtung nach Anspruch 8 bis 16, dadurch gekennzeichnet, daß sie einschließt: Ein Mittel (48, 49) zum Anzeigen von Zahlentabellen, die von Zahlen in den genannten Matrixtabellen abgeleitet werden, in der Art von dreidimensionalen Konturenaufzeichnungen mit der Amplitude der genannten Spektralkomponenten im Vergleich zu Zeit und Frequenz.

**Revendications**

1. Méthode pour l'analyse dans le domaine fréquenciel d'un signal ECG, comprenant les opérations suivantes:- repérer dans un signal ECG un intervalle de temps présentant un intérêt; diviser ledit intervalle de temps présentant un intérêt en une suite de segments ou tranches; multiplier chacune desdites tranches au moyen d'une fonction de fenêtrage pour former une tranche fenêtrée; effectuer une transformation de Fourier rapide (FFT) discrète sur chaque tranche fenêtrée, dans laquelle FFT la densité spectrale de puissance (PSD) à une suite de fréquences est calculée; et produire une matrice numérique ou un tableau de PSD dans lequel des fréquences discrètes désignent chaque colonne dans ledit tableau et des temps discrets désignent chaque ligne dudit tableau, formant ainsi une matrice de PSD pour un groupe de tranches de temps empilées verticalement, caractérisée par le fait que cette méthode comprend les opérations de moyennage de signaux d'un certain nombre de cycles de pulsations cardiaques comprenant ledit signal ECG avant de repérer dans le signal ECG moyenné ledit intervalle de temps présentant un intérêt, celui-ci étant typiquement la portion QRS dudit signal ECG moyenné, et de division dudit intervalle de temps présentant un intérêt en une suite de segments ou tranches se chevauchant.

2. Méthode telle que revendiquée dans la revendication 1, caractérisée par le fait qu'elle comprend les opérations supplémentaires suivantes:- calculer une valeur moyenne de l'amplitude de chaque tranche de temps avant d'effectuer ledit FFT; et soustraire ladite valeur moyenne de chaque tranche de temps de chaque point de mesure dans cette tranche, supprimant ainsi un décalage du niveau continu dans ladite tranche de temps.

3. Méthode telle que revendiquée dans la revendication 1 ou la revendication 2, caractérisée par le fait qu'elle comprend les opérations supplémentaires suivantes:- effectuer les mêmes opérations sur au moins un signal ECG additionnel obtenu d'un patient simultanément avec ledit premier signal ECG, obtenant ainsi une pluralité, N, de signaux ECG et N matrices de PSD; et additionner lesdites valeurs moyennes desdites N matrices de PSD, formant ainsi une (N+1)ième matrice de PSD moyennes.

4. Méthode telle que revendiquée dans n'importe laquelle des revendications précédentes, caractérisée par le fait qu'elle comprend les opérations supplémentaires suivantes: - additionner chaque valeur PSD dans une ligne de tranche de temps pour former une somme de PSD totales, formant ainsi une colonne de PSD totales; et comparer chaque PSD totale dans chaque position verticale dans ladite colonne de PSD avec chaque autre valeur de PSD pour déterminer la ligne de tranche de temps ayant la valeur de PSD totale la plus élevée, et désigner ladite ligne de tranche de temps comme la tranche de temps de foi ou de référence dudit complexe QRS.

5. Méthode telle que revendiquée dans la revendication 4, caractérisée par le fait qu'elle comprend les opérations supplémentaires suivantes:- repérer le début et la fin de la portion QRS dudit signal ECG; calculer la valeur du bruit de fond moyen et l'écart-type dudit signal ECG représenté par ladite matrice de PSD totales en identifiant un intervalle de temps donné, représenté par un nombre pré-déterminé de tranches de temps consécutives dans ladite matrice de PSD ayant les valeurs totales moyennes de PSD les plus basses; et identifier la ligne la plus éloignée (la plus ancienne) et la ligne la plus proche (la plus récente) de ladite tranche de référence du complexe QRS ayant une PSD totale d'au moins NI écarts-types plus grands que ledit bruit de fond moyen.

6. Méthode telle que revendiquée dans la revendication 5, caractérisée par le fait qu'elle comprend l'opération supplémentaire suivante:- calculer la somme des PSD totales dans un intervalle terminal T1 dudit complexe QRS.

7. Méthode telle que revendiquée dans la revendication 6, caractérisée par le fait qu'elle comprend les opérations supplémentaires suivantes:- diviser ladite portion QRS de ladite matrice de PSD en une portion terminale à faible puissance et en une portion à grande puissance, ladite portion terminale à faible puissance étant définie comme commençant à la première tranche de temps suivant ladite tranche de référence de QRS ayant une PSD totale inférieure à un pourcentage P1 de ladite tranche de référence, et se terminant à la fin du complexe QRS, et ladite portion à grande puissance de ladite portion QRS étant définie comme le reste de la portion QRS de ladite matrice de PSD.

8. Appareil pour analyser les signaux ECG pour détecter les anormalités de conduction électrique dans le coeur comprenant: moyen pour enregistrer d'une manière pouvant être sélectionnée une représentation de forme d'onde de la variation de l'amplitude en fonction du temps d'un signal ECG; moyen (4) pour repérer dans ladite forme d'onde un intervalle de temps présentant un intérêt; moyen (8) pour diviser ledit intervalle de temps en une suite de segments ou tranches de temps; moyen (12) pour multiplier chacune desdites tranches par une fonction de fenêtrage pour former une tranche fenêtrée; moyen (16) pour effectuer une transformation de Fourier rapide (FFT) discrète sur chaque tranche fenêtrée; moyen (18) pour calculer la densité spectrale de puissance (PSD) à une série de fréquences dans ladite FFT; et moyen (19) pour produire une matrice numérique ou un tableau de PSD dans lequel des fréquences discrètes désignent chaque colonne dans ledit tableau et des temps discrets désignent chaque ligne dudit tableau, formant ainsi une matrice de PSD pour un groupe de tranches de temps empilées verticalement, caractérisé par le fait que cet appareil comprend un moyen (3) pour effectuer le moyennage de signaux d'un certain nombre de cycles de pulsations cardiaques comprenant ledit signal ECG avant de repérer dans le signal ECG moyenné un intervalle de temps présentant un intérêt, celui-ci étant typiquement la portion QRS dudit signal ECG moyenné, et que ledit moyen (8) de division divise ledit intervalle de temps présentant un intérêt en une suite de segments ou tranches se chevauchant.

9. Appareil tel que revendiqué dans la revendication 8, caractérisé par le fait qu'il comprend des moyens pour comparer ledit tableau de PSD avec des tableaux correspondants obtenus de sujets d'essai normaux et anormaux.

10. Appareil tel que revendiqué dans la revendication 8 ou la revendication 9, caractérisé par le fait qu'il comprend: un moyen (6) pour calculer la valeur moyenne de l'amplitude dans le domaine temporel de chacune desdites tranches de temps, avant d'effectuer ledit fenêtrage; et des moyens (10, 14) pour soustraire ladite valeur moyenne dans le domaine temporel de chaque tranche de chaque valeur de point de mesure dans cette tranche, supprimant ainsi un décalage du niveau continu dans ladite tranche de temps.

11. Appareil tel que revendiqué dans n'importe laquelle des revendications 8 à 10, caractérisé par le fait que ledit appareil est capable d'effectuer les mêmes fonctions sur une pluralité de canaux ECG séparés.

12. Appareil tel que revendiqué dans la revendication 11, caractérisé par le fait qu'il comprend: un moyen (23) pour additionner chaque valeur PSD dans une tranche de temps pour former une somme de PSD totales, formant ainsi une colonne de PSD totales; et un moyen (24) pour comparer ladite valeur de PSD totale dans chaque position verticale dans ladite colonne de PSD avec chaque autre valeur PSD et pour déterminer la ligne de tranche de temps ayant la valeur de PSD totale la plus élevée, déterminant ainsi une tranche de temps de foi ou de référence du complexe QRS desdites formes d'onde ECG.

13. Appareil tel que revendiqué dans la revendication 12, caractérisé par le fait qu'il comprend: un moyen (26) pour repérer le début et la fin de la portion QRS dudit signal ECG; un moyen (27) pour calculer la somme des PSD totales pendant un intervalle terminal T1 dudit complexe QRS; des moyens (28, 29) pour segmenter ladite portion QRS de ladite matrice de PSD en une portion terminale à faible puissance et en une portion à grande puissance, ladite portion terminale à faible puissance étant définie comme commençant à la première tranche de temps suivant ladite tranche QRS ayant une PSD totale inférieure à un pourcentage P1 de ladite tranche de référence, et se terminant à la fin de ladite matrice, et ladite portion à grande puissance de ladite portion QRS étant définie comme le reste de la portion QRS de ladite matrice de PSD.

14. Appareil tel que revendiqué dans la revendication 13, caractérisé par le fait qu'il comprend: un moyen pour un traitement séparé ultérieur comprenant un moyen pour compter les pointes pour les maxima se produisant sur l'axe des fréquences ou les lignes dudit tableau de matrice de PSD (pointes sur l'axe de fréquences), les pointes ou les maxima se produisant sur l'axe de temps ou les colonnes dudit tableau de matrice de PSD (pointes sur l'axe temporel), et les pointes se produisant simultanément sur les deux axes (pointes bi-axiales).

15. Appareil tel que revendiqué dans la revendication 14, caractérisé par le fait qu'il comprend: un moyen (32) pour calculer pour chaque tableau de matrice de PSD susmentionné le coefficient de corrélation Pearson de chaque ligne avec la ligne juste en dessous (coefficient de corrélation entre tranches) et pour enregistrer pour l'afficher une colonne supplémentaire de chiffres correspondant auxdits coefficients de corrélation.

16. Appareil tel que revendiqué dans la revendication 15, caractérisé par le fait qu'il comprend: un moyen (35) pour calculer et enregistrer pour l'afficher une valeur appelée entropie spectrale en ajoutant une ligne supplémentaire à la table de matrice de PSD, ladite ligne supplémentaire contenant la PSD moyenne calculée de chaque fréquence harmonique des lignes sélectionnées desdites PSD en calculant la corrélation moyenne de chaque ligne de tranche de temps avec la nouvelle ligne de PSD moyennes et en soustrayant ladite corrélation moyenne de 1.

17. Appareil tel que revendiqué dans n'importe laquelle des revendications 8 à 16, caractérisé par le fait qu'il comprend: des moyens (48, 49) pour afficher les tableaux de chiffres tirés de chiffres dans lesdits tableaux de matrices dans des tracés de courbes de niveau de type tridimensionnel avec l'amplitude desdites composantes spectrales tracées en fonction du temps et de la fréquence.

FIG. 1a

EP 0 448 196 B1

FIG. 1b

FROM FIG. 1a

19 — LEAD X PSD MATRIX

23 — SUMMER OF PSD's FOR EACH SLICE

24 — FIDUCIAL SLICE IDENTIFICATION

25 — NOISE MEAN & STAND. DEV. CALCULATOR

26 — QRS ONSET AND OFFSET LOCATOR

28 — LOW POWER REGION LOCATOR

30 — PEAK COUNTER

31 — INTERSLICE CORRELATION CALCULATOR

32 — LOW SLICE CORRELATION COUNTER

27 — PSD 40 msec CALCULATOR

35 — SPECTRAL ENTROPY COUNTER

29 — HIGH POWER REGION LOCATOR

33 — INTERSLICE CORRELATION MEAN AND STAND. DEV. CALCULATOR

X+Y+Z PSD MATRIX

22

20 — LEAD Y PSD MATRIX

34 — INTERSLICE CORRELATION SKEW

21 — LEAD Z PSD MATRIX

39 — X+Y+Z DATA REGISTER

36 — NUMERICAL DATA STORAGE REGISTER, LEAD X

360 — NUMERIC PRINTED REPORT

37 — LEAD Y DATA REGISTER

38 — LEAD Z DATA REGISTER

DEFAULT: OMITTED 47

SCALE COMPRESSOR 46

PLOTTER (LASER PRINTER) 48

DISPLAY (COLOR CRT) 49

DEFAULT: PRINT ALL VIEWS, DISPLAY "BIRDS EYE" 45

MAP ORIENTATION SELECTOR 44

DEFAULT: FULLSCALE = 5% PEAK POWER 43

GAIN SELECTOR 42

DEFAULT: OMITTED 41

POST-PROCESSOR 40

LEAD X PSD MATRIX 19

LEAD Y PSD MATRIX 20

LEAD Z PSD MATRIX 21

X+Y+Z PSD MATRIX 22

FROM FIG. 1a

FROM FIG. 1b

NORMAL VOLUNTEER—LATE POTENTIALS ABSENT—SMOOTH SPECTRAL CONTOURS
—LOW SPECTRAL TURBULENCE

FIG. 2

Spectrocardiogram is a trademark of Del Mar Avionics

EP 0 448 196 B1

EP 0 448 196 B1

NORMAL VOLUNTEER WITH MINOR NORMAL VARIANT ON ECG
"FALSE POSITIVE" LATE POTENTIALS BUT LOW SPECTRAL TURBULENCE

Patient:
Age:          Sex:
Indication:
Physician:
Medication:
Comment:

Report No. :
Report Date:
Test Date:  11/16/87
Sample Time: 14:43

Beats Averaged:    402
Sampling Frequency:  1000 Hz
File Name:        beckerb.r00

T1          T2

---

LEAD X

Spectrocardiogram™        Parameters:

Time slice duration:      24.0 msec.
Time slice step interval:  2.0 msec.
Signal preconditioning:    Velocity
DC Offset subtraction:    Pre-Window
FFT Points:              64
Window:              Blackman-Harris

PSD %
5.00
4.50
4.00
3.50
3.00
2.50
2.00
1.50
1.00
0.50

LATE POTENTIALS PRESENT

T1
45.6
91.2
136.8
T2    Time (msec.)

0    250    500
Frequency (Hz)

---

LEAD X

Spectrocardiogram™        Parameters:

Time slice duration:      24.0 msec.
Time slice step interval:  2.0 msec.
Signal preconditioning:    Velocity
DC Offset subtraction:    Pre-Window
FFT Points:              64
Window:              Blackman-Harris

PSD %
100
90
80
70
60
50
40
30
20
10
0 Hz
250 Hz
500 Hz

PSD %
5.00
4.50
4.00
3.50
3.00
2.50
2.00
1.50
1.00
0.50
0 Hz
250 Hz
500 Hz

LATE POTENTIALS PRESENT

T1    46    91    137    182    T2
Time (msec.)

---

LEAD X

Spectrocardiogram™        Parameters:

Time slice duration:      24.0 msec.
Time slice step interval:  2.0 msec.
Signal preconditioning:    Velocity
DC Offset subtraction:    Pre-Window
FFT Points:              64
Window:              Blackman-Harris

PSD %
5.00
4.50
4.00
3.50
3.00
2.50
2.00
1.50
1.00
0.50

T1
45.6
91.2
136.8
T2

0    250    500
Frequency (Hz)

---

LEAD X

Spectrocardiogram™        Parameters:

Time slice duration:      24.0 msec.
Time slice step interval:  2.0 msec.
Signal preconditioning:    Velocity
DC Offset subtraction:    Pre-Window
FFT Points:              64
Window:              Blackman-Harris

PSD %
5.00
4.50
4.00
3.50
3.00
2.50
2.00
1.50
1.00
0.50

SMOOTH SPECTRAL CONTOURS,
MUCH WHITE SPACE

T1
45.6
91.2
136.8
T2

0    250    500
Frequency (Hz)

---

Spectrocardiogram is a trademark of Del Mar Avionics

FIG. 3

PATIENT AT RISK OF ARRHYTHMIA   LATE POTENTIALS PRESENT- "TRUE POSITIVE"
HIGH SPECTRAL TURBULENCE

FIG. 4

Spectrocardiogram is a trademark of Del Mar Avionics

| Patient: | | Report No. : | | Beats Averaged: 322 | Spectrocardiogram[TM] | Parameters: |
|---|---|---|---|---|---|---|
| Age: | Sex: | Report Date: | | Sampling Frequency: 1000 Hz | Time slice duration: | 24.0 msec. |
| Indication: | | Test Date: 02/08/88 | | File Name: a.r00 | Time slice step interval: | 2.0 msec. |
| Physician: | | Sample Time: 14:23 | | | Signal preconditioning: | Velocity |
| Medication: | | | | | DC Offset subtraction: | Pre-Window |
| Comment: | | | | | FFT Points: | 64 |
| | | | | | Window: | Blackman-Harris |

| LEAD X | | LEAD Y | | LEAD Z | | X + Y + Z | |
|---|---|---|---|---|---|---|---|
| Total QRS Duration: | 88.0 msec | Total QRS Duration: | 98.0 msec | Total QRS Duration: | 120.0 msec | Total QRS Duration: | 106.0 msec |

**LOW POWER TERMINAL QRS REGION**

| LEAD X | | LEAD Y | | LEAD Z | | X + Y + Z | |
|---|---|---|---|---|---|---|---|
| PSD40: | 12.0 msec | PSD40: | 20.0 msec | PSD40: | 36.0 msec | PSD40: | 24.0 msec |
| HFQRS Duration: | 77.3 | HFQRS Duration: | 107.3 | HFQRS Duration: | 10.7 | HFQRS Duration: | 37.1 |
| HF Biaxial Peaks: | 0 | HF Biaxial Peaks: | 2 | HF Biaxial Peaks: | 1 | HF Biaxial Peaks: | 0 |
| HF Frequency Axis Peaks: | 5 | HF Frequency Axis Peaks: | 5 | HF Frequency Axis Peaks: | 13 | HF Frequency Axis Peaks: | 11 |
| HF Temporal Axis Peaks: | 8 | HF Temporal Axis Peaks: | 9 | HF Temporal Axis Peaks: | 10 | HF Temporal Axis Peaks: | 6 |
| LF Biaxial Peaks: | 0 | LF Biaxial Peaks: | 0 | LF Biaxial Peaks: | 0 | LF Biaxial Peaks: | 0 |
| LF Frequency Axis Peaks: | 0 | LF Frequency Axis Peaks: | 0 | LF Frequency Axis Peaks: | 0 | LF Frequency Axis Peaks: | 0 |
| LF Temporal Axis Peaks: | 0 | LF Temporal Axis Peaks: | 0 | LF Temporal Axis Peaks: | 0 | LF Temporal Axis Peaks: | 0 |
| Spectral Turbulence: | 2 | Spectral Turbulence: | 5 | Spectral Turbulence: | 3 | Spectral Turbulence: | 0 |
| Late Potential Duration: | 5.7 | Late Potential Duration: | 4.8 | Late Potential Duration: | 5.9 | Late Potential Duration: | 1.8 |

**HIGH POWER MAIN QRS REGION**

| LEAD X | | LEAD Y | | LEAD Z | | X + Y + Z | |
|---|---|---|---|---|---|---|---|
| Region Duration: | 70.0 msec | Region Duration: | 76.0 msec | Region Duration: | 84.0 msec | Region Duration: | 80.0 msec |
| HF Biaxial Peaks: | 6 | HF Biaxial Peaks: | 9 | HF Biaxial Peaks: | 8 | HF Biaxial Peaks: | 6 |
| HF Frequency Axis Peaks: | 38 | HF Frequency Axis Peaks: | 63 | HF Frequency Axis Peaks: | 60 | HF Frequency Axis Peaks: | 43 |
| HF Temporal Axis Peaks: | 75 | HF Temporal Axis Peaks: | 113 | HF Temporal Axis Peaks: | 105 | HF Temporal Axis Peaks: | 91 |
| LF Biaxial Peaks: | 0 | LF Biaxial Peaks: | 0 | LF Biaxial Peaks: | 0 | LF Biaxial Peaks: | 0 |
| LF Frequency Axis Peaks: | 0 | LF Frequency Axis Peaks: | 0 | LF Frequency Axis Peaks: | 0 | LF Frequency Axis Peaks: | 0 |
| LF Temporal Axis Peaks: | 0 | LF Temporal Axis Peaks: | 0 | LF Temporal Axis Peaks: | 0 | LF Temporal Axis Peaks: | 0 |
| Spectral Turbulence: | 7 | Spectral Turbulence: | 13 | Spectral Turbulence: | 13 | Spectral Turbulence: | 6 |
| Late Potential Duration: | 10.1 | Late Potential Duration: | 22.5 | Late Potential Duration: | 23.8 | Late Potential Duration: | 9.8 |

INTERPRETATION:

*FIG. 5*

# "FALSE POSITIVE" HEALTHY VOLUNTEER

| Patient: | Report No. : | Beats Averaged: 402 |
|---|---|---|
| Age: Sex: | Report Date: | Sampling Frequency: 1000 Hz |
| Indication: | Test Date: 11/16/87 | File Name: beckerb.r00 |
| Physician: | Sample Time: 14:43 | |
| Medication: | | |
| Comment: | | |

| Spectrocardiogram[TM] | Parameters: |
|---|---|
| Time slice duration: | 24.0 msec. |
| Time slice step interval: | 2.0 msec. |
| Signal preconditioning: | Velocity |
| DC Offset subtraction: | Pre-Window |
| FFT Points: | 64 |
| Window: | Blackman-Harris |

### LEAD X
Total QRS Duration:  92.0 msec

**LOW POWER TERMINAL QRS REGION**

PSD40:  18.0 msec
HFQRS Duration:  134.9

HF Biaxial Peaks:  3
HF Frequency Axis Peaks:  8
HF Temporal Axis Peaks:  10

LF Biaxial Peaks:  0
LF Frequency Axis Peaks:  0
LF Temporal Axis Peaks:  0

Spectral Turbulence:  8
Late Potential Duration: 13.9

**HIGH POWER MAIN QRS REGION**

Region Duration:  66.0 msec

HF Biaxial Peaks:  4
HF Frequency Axis Peaks:  53
HF Temporal Axis Peaks:  96

LF Biaxial Peaks:  0
LF Frequency Axis Peaks:  0
LF Temporal Axis Peaks:  0

Spectral Turbulence:  7
Late Potential Duration: 11.5

### LEAD Y
Total QRS Duration:  124.0 msec

**LOW POWER TERMINAL QRS REGION**

PSD40:  52.0 msec
HFQRS Duration:  0.3

HF Biaxial Peaks:  4
HF Frequency Axis Peaks:  23
HF Temporal Axis Peaks:  24

LF Biaxial Peaks:  0
LF Frequency Axis Peaks:  0
LF Temporal Axis Peaks:  0

Spectral Turbulence:  11
Late Potential Duration: 17.9

**HIGH POWER MAIN QRS REGION**

Region Duration:  66.0 msec

HF Biaxial Peaks:  8
HF Frequency Axis Peaks:  58
HF Temporal Axis Peaks:  107

LF Biaxial Peaks:  0
LF Frequency Axis Peaks:  0
LF Temporal Axis Peaks:  0

Spectral Turbulence:  12
Late Potential Duration: 20.7

### LEAD Z
Total QRS Duration:  130.0 msec

**LOW POWER TERMINAL QRS REGION**

PSD40:  52.0 msec
HFQRS Duration:  1.5

HF Biaxial Peaks:  3
HF Frequency Axis Peaks:  19
HF Temporal Axis Peaks:  30

LF Biaxial Peaks:  0
LF Frequency Axis Peaks:  0
LF Temporal Axis Peaks:  0

Spectral Turbulence:  10
Late Potential Duration: 16.0

**HIGH POWER MAIN QRS REGION**

Region Duration:  78.0 msec

HF Biaxial Peaks:  10
HF Frequency Axis Peaks:  68
HF Temporal Axis Peaks:  118

LF Biaxial Peaks:  0
LF Frequency Axis Peaks:  0
LF Temporal Axis Peaks:  0

Spectral Turbulence:  12
Late Potential Duration: 21.0

### X + Y + Z
Total QRS Duration:  122.0 msec

**LOW POWER TERMINAL QRS REGION**

PSD40:  46.0 msec
HFQRS Duration:  1.4

HF Biaxial Peaks:  5
HF Frequency Axis Peaks:  23
HF Temporal Axis Peaks:  19

LF Biaxial Peaks:  0
LF Frequency Axis Peaks:  0
LF Temporal Axis Peaks:  0

Spectral Turbulence:  7
Late Potential Duration:  6.0

**HIGH POWER MAIN QRS REGION**

Region Duration:  72.0 msec

HF Biaxial Peaks:  4
HF Frequency Axis Peaks:  54
HF Temporal Axis Peaks:  100

LF Biaxial Peaks:  0
LF Frequency Axis Peaks:  0
LF Temporal Axis Peaks:  0

Spectral Turbulence:  6
Late Potential Duration:  9.1

INTERPRETATION:

# FIG. 6

EP 0 448 196 B1

## "TRUE POSITIVE" PATIENT AT RISK

| | |
|---|---|
| Patient: | |
| Age:        Sex: | |
| Indication: | |
| Physician: | |
| Medication: | |
| Comment: | |

| |
|---|
| Report No. :0687 |
| Report Date: |
| Test Date:   29.08.08 |
| Sample Time:05:15 |

| |
|---|
| Beats Averaged:    675 |
| Sampling Frequency:  1000 Hz |
| File Name:        berry.r00 |

Spectrocardiogram™  Parameters:

| | |
|---|---|
| Time slice duration: | 24.0 msec. |
| Time slice step interval: | .2.0 msec. |
| Signal preconditioning: | Velocity |
| DC Offset subtraction: | Pre-Window |
| FFT Points: | 64 |
| Window: | Blackman-Harris |

### LEAD X
Total QRS Duration:     166.0 msec

### LEAD Y
Total QRS Duration:     170.0 msec

### LEAD Z
Total QRS Duration:     140.0 msec

### X + Y + Z
Total QRS Duration:     164.0 msec

---

**LOW POWER TERMINAL QRS REGION**

| | |
|---|---|
| PSD40: | 70.0 msec |
| HFQRS Duration: | 1.8 |
| HF Biaxial Peaks: | 5 |
| HF Frequency Axis Peaks: | 40 |
| HF Temporal Axis Peaks: | 69 |
| LF Biaxial Peaks: | 0 |
| LF Frequency Axis Peaks: | 0 |
| LF Temporal Axis Peaks: | 0 |
| Spectral Turbulence: | 18 |
| Late Potential Duration: | 33.0 |

**LOW POWER TERMINAL QRS REGION**

| | |
|---|---|
| PSD40: | 72.0 msec |
| HFQRS Duration: | 0.6 |
| HF Biaxial Peaks: | 7 |
| HF Frequency Axis Peaks: | 41 |
| HF Temporal Axis Peaks: | 59 |
| LF Biaxial Peaks: | 0 |
| LF Frequency Axis Peaks: | 0 |
| LF Temporal Axis Peaks: | 0 |
| Spectral Turbulence: | 26 |
| Late Potential Duration: | 47.0 |

**LOW POWER TERMINAL QRS REGION**

| | |
|---|---|
| PSD40: | 44.0 msec |
| HFQRS Duration: | 3.0 |
| HF Biaxial Peaks: | 5 |
| HF Frequency Axis Peaks: | 28 |
| HF Temporal Axis Peaks: | 56 |
| LF Biaxial Peaks: | 0 |
| LF Frequency Axis Peaks: | 0 |
| LF Temporal Axis Peaks: | 0 |
| Spectral Turbulence: | 12 |
| Late Potential Duration: | 18.3 |

**LOW POWER TERMINAL QRS REGION**

| | |
|---|---|
| PSD40: | 68.0 msec |
| HFQRS Duration: | 1.1 |
| HF Biaxial Peaks: | 5 |
| HF Frequency Axis Peaks: | 35 |
| HF Temporal Axis Peaks: | 56 |
| LF Biaxial Peaks: | 0 |
| LF Frequency Axis Peaks: | 0 |
| LF Temporal Axis Peaks: | 0 |
| Spectral Turbulence: | 13 |
| Late Potential Duration: | 21.0 |

---

**HIGH POWER MAIN QRS REGION**

| | |
|---|---|
| Region Duration: | 96.0 msec |
| HF Biaxial Peaks: | 12 |
| HF Frequency Axis Peaks: | 85 |
| HF Temporal Axis Peaks: | 155 |
| LF Biaxial Peaks: | 0 |
| LF Frequency Axis Peaks: | 0 |
| LF Temporal Axis Peaks: | 0 |
| Spectral Turbulence: | 16 |
| Late Potential Duration: | 25.9 |

**HIGH POWER MAIN QRS REGION**

| | |
|---|---|
| Region Duration: | 94.0 msec |
| HF Biaxial Peaks: | 15 |
| HF Frequency Axis Peaks: | 76 |
| HF Temporal Axis Peaks: | 145 |
| LF Biaxial Peaks: | 0 |
| LF Frequency Axis Peaks: | 0 |
| LF Temporal Axis Peaks: | 0 |
| Spectral Turbulence: | 26 |
| Late Potential Duration: | 49.2 |

**HIGH POWER MAIN QRS REGION**

| | |
|---|---|
| Region Duration: | 92.0 msec |
| HF Biaxial Peaks: | 10 |
| HF Frequency Axis Peaks: | 70 |
| HF Temporal Axis Peaks: | 132 |
| LF Biaxial Peaks: | 0 |
| LF Frequency Axis Peaks: | 0 |
| LF Temporal Axis Peaks: | 0 |
| Spectral Turbulence: | 20 |
| Late Potential Duration: | 26.9 |

**HIGH POWER MAIN QRS REGION**

| | |
|---|---|
| Region Duration: | 96.0 msec |
| HF Biaxial Peaks: | 9 |
| HF Frequency Axis Peaks: | 71 |
| HF Temporal Axis Peaks: | 138 |
| LF Biaxial Peaks: | 0 |
| LF Frequency Axis Peaks: | 0 |
| LF Temporal Axis Peaks: | 0 |
| Spectral Turbulence: | 6 |
| Late Potential Duration: | 11.6 |

INTERPRETATION:

## FIG. 7

EP 0 448 196 B1

EP 0 448 196 B1

| Patient: | Report No. : | Beats Averaged: 322 |
| Age: Sex: | Report Date: | Sampling Frequency: 1000 Hz |
| Indication: | Test Date: 02/08/88 | File Name: x.r00 |
| Physician: | Sample Time: 14:23 | |
| Medication: | | |
| Comment: | | |

**LEAD X**

Spectrocardiogram[TM]

| Time slice duration: | 24.0 msec. |
| Time slice step interval: | 2.0 msec. |
| Signal preconditioning: | Velocity |
| DC Offset subtraction: | Pre-Window |
| FFT Points: | 64 |
| Window: | Blackman-Harris |

Parameters:

PSD %

5.00, 4.50, 4.00, 3.50, 3.00, 2.50, 2.00, 1.50, 1.00, 0.50

T1, 51.2, 102.4, 153.6, 204.8, T2, Time (msec.)

0 250 500
Frequency (Hz)

**LEAD Y**

Spectrocardiogram[TM]

| Time slice duration: | 24.0 msec. |
| Time slice step interval: | 2.0 msec. |
| Signal preconditioning: | Velocity |
| DC Offset subtraction: | Pre-Window |
| FFT Points: | 64 |
| Window: | Blackman-Harris |

Parameters:

PSD %

5.00, 4.50, 4.00, 3.50, 3.00, 2.50, 2.00, 1.50, 1.00, 0.50

T1, 51.2, 102.4, 153.6, 204.8, T2, Time (msec.)

0 250 500
Frequency (Hz)

**LEAD Z**

Spectrocardiogram[TM]

| Time slice duration: | 24.0 msec. |
| Time slice step interval: | 2.0 msec. |
| Signal preconditioning: | Velocity |
| DC Offset subtraction: | Pre-Window |
| FFT Points: | 64 |
| Window: | Blackman-Harris |

Parameters:

PSD %

5.00, 4.50, 4.00, 3.50, 3.00, 2.50, 2.00, 1.50, 1.00, 0.50

T1, 51.2, 102.4, 153.6, 204.8, T2, Time (msec.)

0 250 500
Frequency (Hz)

**Sum (X + Y + Z)**

Spectrocardiogram[TM]

| Time slice duration: | 24.0 msec. |
| Time slice step interval: | 2.0 msec. |
| Signal preconditioning: | Velocity |
| DC Offset subtraction: | Pre-Window |
| FFT Points: | 64 |
| Window: | Blackman-Harris |

Parameters:

PSD %

5.00, 4.50, 4.00, 3.50, 3.00, 2.50, 2.00, 1.50, 1.00, 0.50

T1, 51.2, 102.4, 153.6, 204.8, T2, Time (msec.)

0 250 500
Frequency (Hz)

Spectrocardiogram is a trademark of Del Mar Avionics

## FIG. 8

EP 0 448 196 B1

Patient:
Age:          Sex:
Indication:
Physician:
Medication:
Comment:

Report No. :
Report Date:
Test Date:   02/08/88
Sample Time: 14:23

Beats Averaged:      322
Sampling Frequency:  1000 Hz
File Name:      a.r00

T1          T2

LEAD X

Spectrocardiogram[TM]          Parameters:

Time slice duration:           24.0 msec.
Time slice step interval:       2.0 msec.
Signal preconditioning:        Velocity
DC Offset subtraction:         Pre-Window
FFT Points:                    128
Window:                        Blackman-Harris

Log (dB)

T1
51.2
102.4
153.6
204.8
T2   Time (msec.)

0   250   500
Frequency (Hz)

LEAD Y

Spectrocardiogram[TM]          Parameters:

Time slice duration:           24.0 msec.
Time slice step interval:       2.0 msec.
Signal preconditioning:        Velocity
DC Offset subtraction:         Pre-Window
FFT Points:                    128
Window:                        Blackman-Harris

Log (dB)

T1
51.2
102.4
153.6
204.8
T2   Time (msec.)

0   250   500
Frequency (Hz)

LEAD Z

Spectrocardiogram[TM]          Parameters:

Time slice duration:           24.0 msec.
Time slice step interval:       2.0 msec.
Signal preconditioning:        Velocity
DC Offset subtraction:         Pre-Window
FFT Points:                    128
Window:                        Blackman-Harris

Log (dB)

T1
51.2
102.4
153.6
204.8
T2   Time (msec.)

0   250   500
Frequency (Hz)

Sum (X + Y + Z)

Spectrocardiogram[TM]          Parameters:

Time slice duration:           24.0 msec.
Time slice step interval:       2.0 msec.
Signal preconditioning:        Velocity
DC Offset subtraction:         Pre-Window
FFT Points:                    128
Window:                        Blackman-Harris

Log (dB)

T1
51.2
102.4
153.6
204.8
T2   Time (msec.)

0   250   500
Frequency (Hz)

Spectrocardiogram is a trademark of Del Mar Avionics

FIG. 9

EXAMPLE PSD MATRIX TABLE,
ONE FOR EACH LEAD, AS
WELL AS X+Y+Z

K SLICES, N POINT FFT, $F_0$ IS FUNDAMENTAL-
$F_X$ IS $X^{TH}$ HARMONIC

FIG. 10